(19) Europäisches Patentamt
European Patent Office
Office européen des brevets

(11) EP 1 944 295 A1

(12) EUROPEAN PATENT APPLICATION

(43) Date of publication:
16.07.2008 Bulletin 2008/29

(51) Int Cl.:
*C07D 231/06* (2006.01) *A61K 31/415* (2006.01)
*A61P 3/06* (2006.01)

(21) Application number: 07384005.0

(22) Date of filing: 15.01.2007

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK RS

(71) Applicant: Laboratorios del Dr. Esteve S.A.
08041 Barcelona (ES)

(72) Inventors:
- Buschmann, Helmut
08960 Sant Just Desvern (ES)
- Torrens-Jover, Antonio
08221 Terrassa (Barcelona) (ES)

(74) Representative: Peters, Hajo et al
Bosch Graf von Stosch Jehle
Patentanwaltsgesellschaft mbH
Flüggenstrasse 13
80639 München (DE)

# (54) Non-racemic mixtures of (R)-N-piperidinyl-5-(4-chlorophenyl)1-(2,4-dichlorophenyl)-4,5-dihydro-1H-pyrazole-3-carboxamide and (S)-N-piperidinyl-5-(4-chlorophenyl)-1-(2,4-dichlorophenyl)-4,5-dihydro-1H-pyrazole-3-carboxamide

(57) The present invention relates to non-racemic mixtures of (R)-N-piperidinyl-5-(4-chlorophenyl)-1-(2,4-dichlorophenyl)-4,5-dihydro-1H-pyrazole-3-carboxamide and (S)-N-piperidinyl-5-(4-chlorophenyl)-1-(2,4-dichlorophenyl)-4,5-dihydro-1 H-pyrazole-3-carboxamide, methods for their preparation, medicaments comprising the mixtures as well as use for the preparation of a medicament for the treatment of humans and animals and the use of (S)-N-piperidinyl-5-(4-chlorophenyl)-1-(2,4-dichlorophenyl)-4,5-dihydro-1H-pyrazole-3-carboxamide in the prophylaxis and/or treatment of dyslipidaemia and other metabolic disturbances that result from the excessive consumption of highly palatable macronutrient food sources.

EP 1 944 295 A1

## Description

The present invention relates to non-racemic mixtures of (R)-N-piperidinyl-5-(4-chlorophenyl)-1-(2,4-dichlorophenyl)-4,5-dihydro-1H-pyrazole-3-carboxamide and (S)-N-piperidinyl-5-(4-chlorophenyl)-1-(2,4-dichlorophenyl)-4,5-dihydro-1H-pyrazole-3-carboxamide, methods for their preparation, medicaments comprising the mixtures as well as use for the preparation of a medicament for the treatment of humans and animals and the use of (S)-N-piperidinyl-5-(4-chlorophenyl)-1-(2,4-dichlorophenyl)-4,5-dihydro-1H-pyrazole-3-carboxamide in the prophylaxis and/or treatment of dyslipidaemia and other metabolic disturbances that result from the excessive consumption of highly palatable macronutrient food sources.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

It is now abundantly clear that abundant access to highly palatable, calorie-dense, low-cost Westernised diets is producing an explosion in the prevalence of dyslipidaemia, obesity, particularly central adiposity, insulin resistance/impaired glucose tolerance and/or Type 2 diabetes (Zephier et al, 1997; International Diabetes Federation, 2003; Grundy, 2004; Deedwania, 2004). Moreover, when occurring in combination, these cardio-metabolic risk factors comprise the Metabolic Syndrome as defined for example by expert bodies, eg World Health Organisation (WHO, 1999) and the National Cholesterol Education Programme - Third Adult Treatment Panel (NCEP ATP III, 2001) and the International Diabetes Federation (IDF, 2005). In addition, there are subjects, for whom their susceptibility to becoming overweight or obese and to suffer obesity-related metabolic disorders with their associated risks of premature morbidity and mortality, is related not to the over-consumption of a balanced diet; rather it is driven by the excessive consumption, and in some instances "bingeing" on specific macronutrient sources, eg "carbohydrate craving" for sweets, chocolate, biscuits and cakes, or high-fat processed sources, including hamburgers, chips and pies, or high protein sources including excessive consumption of red meat (White et al, 2002; Phelan & Whadden, 2002; Yanovski, 2003; White & Grilo, 2005).

Thus, it was an object of the present invention to provide novel ways related to compounds or mixtures of compounds for use as active substances in medicaments in the prophylaxis and/or treatment of obesity, diabetes type II, dyslipidaemia, or metabolic syndrome. A very high focus was given to the prophylaxis and/or treatment of dyslipidaemia and other metabolic disturbances that result from the excessive consumption of highly palatable macronutrient food sources.

Nevertheless, as it has to be recognized, that the patient population suffering from these symptoms or the group of persons taking care to prevent the occurance of these symptoms is or can be highly diverse with some subgroups being obese or overweight, some groups being on the brink of that and some groups having normal weight. Thus it was also part of the object of the present invention to provide a more suitable and tailored approach of treatment/prophylaxis to adapt more to specific needs of these heterogeneous groups.

Said object was achieved by providing the non-racemic mixture of the piperidinyl-pyrazoline compounds given below.

Thus, in one of its aspects the present invention relates to the non-racemic mixture of (S)-N-piperidinyl-5-(4-chlorophenyl)-1-(2,4-dichlorophenyl)-4,5-dihydro-1H-pyrazole-3-carboxamide and (R)-N-piperidinyl-5-(4-chlorophenyl)-1-(2,4-dichlorophenyl)-4,5-dihydro-1H-pyrazole-3-carboxamide, their respective salts, polymorphs or solvates, especially hydrates.

It has been found that one of the 2 compounds of this non-racemic mixture according to the invention, (R)-N-piperidinyl-5-(4-chlorophenyl)-1-(2,4-dichlorophenyl)-4,5-dihydro-1H-pyrazole-3-carboxamide (hereinafter also called Compound A), has a high affinity for cannabinoid receptors, particularly for the $CB_1$-receptor, and that it acts as modulators

e.g. antagonists, inverse agonists or agonists on these receptors. The non-racemic mixture according to the invention therefore is suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Even though (S)-N-piperidinyl-5-(4-chlorophenyl)-1-(2,4-dichlorophenyl)-4,5-dihydro-1 H-pyrazole-3-carboxamide (hereinafter also called Compound B) lacks pharmacologically-relevant affinity for the CB1-receptor in rats it has been surprisingly shown that (S)-N-piperidinyl-5-(4-chlorophenyl)-1-(2,4-dichlorophenyl)-4,5-dihydro-1H-pyrazole-3-carboxamide reduces the craving for and over-consumption of highly palatable, calorie-dense macronutrient food sources, also the resulting obesity and metabolic disturbances of dyslipidaemia and insulin resistance/impaired glucose tolerance. As shown later, high fat foods have been exemplified by high-fat chow, carbohydrate foods have been exemplified by ground dairy milk chocolate and savoury high protein foods have been exemplified by ground roasted salted peanuts; all were freely available to the animals as individual "cafeteria" macronutrient sources. Reductions in the excessive consumption of highly palatable food sources evoked by (S)-N-piperidinyl-5-(4-chlorophenyl)-1-(2,4-dichlorophenyl)-4,5-dihydro-1 H-pyrazole-3-carboxamide produce the therapeutic benefits of decreased body weight, reduced adiposity, decreased plasma concentrations of non-esterified fatty acids (NEFAs; or free fatty acids [FFAs]), triglycerides (triacylglycerol and glycerol) and insulin.

These findings are unexpected for 3 reasons. First, as described in literature antagonists or inverse agonists of cannabinoid CB1 receptors and these respective drugs produce their beneficial metabolic effects through this molecular target; since COMPOUND B lacks pharmacologically-relevant affinity for this receptor in the rat CB1 receptor, in which the experiments were done, its therapeutic effects on body weight, adiposity, and plasma concentrations of NEFAs, triglycerides and insulin are consequently independent of the blockade of central or peripheral CB1 receptors. Second, the improvements in plasma lipid profiles produced by COMPOUND B treatment are greater than those that can be explained on the basis of weight reduction, demonstrating that COMPOUND B has weight-loss-independent benefits as a compound to treat dyslipidaemia. Third, COMPOUND B also unexpectedly reduced plasma concentrations of NEFAs, and as they are key drivers of the progression from insulin resistance/impaired glucose tolerance (pre-diabetes) to Type 2 diabetes (Boden & Laakso, 2004; Bays et al, 2004; IDF, 2005), this unexpected finding demonstrates that COMPOUND B will also have benefits in the management of pre-diabetes and Type 2 diabetes. Collectively, these surprising beneficial effects of COMPOUND B on abnormalities of body weight, adiposity, and plasma concentrations of NEFAs, triglycerides and insulin resulting from excessive consumption of or bingeing on highly palatable macronutrient food sources demonstrate that this compound will also be useful as a medicament for the treatment and/or prophylaxis of Metabolic Syndrome. As (S)-N-piperidinyl-5-(4-chlorophenyl)-1-(2,4-dichlorophenyl)-4,5-dihydro-1H-pyrazole-3-carboxamide (COMPOUND B) was demonstrating its beneficial effects also in part weight-loss-independent while showing only a more mild loss of body weight than (R)-N-piperidinyl-5-(4-chlorophenyl)-1-(2,4-dichlorophenyl)-4,5-dihydro-1H-pyrazole-3-carboxamide (Compound A; see below) non-racemic mixtures of Compound A and B might be tailored to the needs of an individual (e.g. more Compound B in an individual, not being obese but under risk needing prevention or treatment, or the opposite with a person being slightly overweight).

"Non-racemic" according to the invention is defined as meaning any mixture that is not exactly 1:1.

As preferred embodiment of the present invention in the non-racemic mixture according to the invention (S)-N-piperidinyl-5-(4-chlorophenyl)-1-(2,4-dichlorophenyl)-4,5-dihydro-1H-pyrazole-3-carboxamide is present in the mixture in an amount X of between 99 to 51 percent by weight, preferably 95 to 55 percent by weight, more preferably 90 to 60 percent by weight, most preferably 80 to 60 percent by weight and
(R)-N-piperidinyl-5-(4-chlorophenyl)-1-(2,4-dichlorophenyl)-4,5-dihydro-1H-pyrazole-3-carboxamide is present in the mixture in an amount Y of between 01 to 49 percent by weight, preferably 05 to 45 percent by weight, more preferably 10 to 40 percent by weight, most preferably 20 to 40 percent by weight,
with X+Y being 100.

As another preferred embodiment of the present invention in the non-racemic mixture according to the invention (R)-N-piperidinyl-5-(4-chlorophenyl)-1-(2,4-dichlorophenyl)-4,5-dihydro-1H-pyrazole-3-carboxamide is present in the mixture in an amount X of between 99 to 51 percent by weight, preferably 95 to 55 percent by weight, more preferably 90 to 60 percent by weight, most preferably 80 to 60 percent by weight and
(S)-N-piperidinyl-5-(4-chlorophenyl)-1-(2,4-dichlorophenyl)-4,5-dihydro-1H-pyrazole-3-carboxamide is present in the mixture in an amount Y of between 01 to 49 percent by weight, preferably 05 to 45 percent by weight, more preferably 10 to 40 percent by weight, most preferably 20 to 40 percent by weight,
with X+Y being 100.

As another preferred embodiment of the present invention in the nonracemic mixture according to the invention one of (S)-N-piperidinyl-5-(4-chlorophenyl)-1-(2,4-dichlorophenyl)-4,5-dihydro-1H-pyrazole-3-carboxamide or (R)-N-piperidinyl-5-(4-chlorophenyl)-1-(2,4-dichlorophenyl)-4,5-dihydro-1H-pyrazole-3-carboxamide is present in in form of its base or hydrochloride, preferably both are in form of their respective bases or hydrochlorides, most preferably both are in form of their respective bases.

(R)-N-piperidinyl-5-(4-chlorophenyl)-1-(2,4-dichlorophenyl)-4,5-dihydro-1H-pyrazole-3-carboxamide (formula Ia) may be prepared with a compound of formula IIa

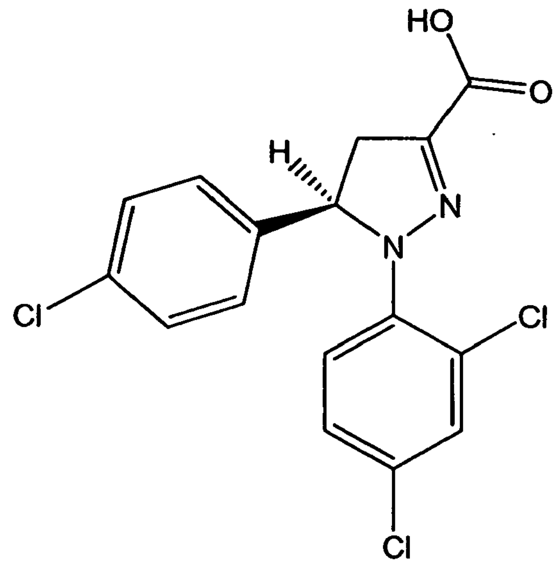

IIa

, which is optionally transferred under inert atmosphere to a compound of general formula (IIIa) via reaction with an activating agent

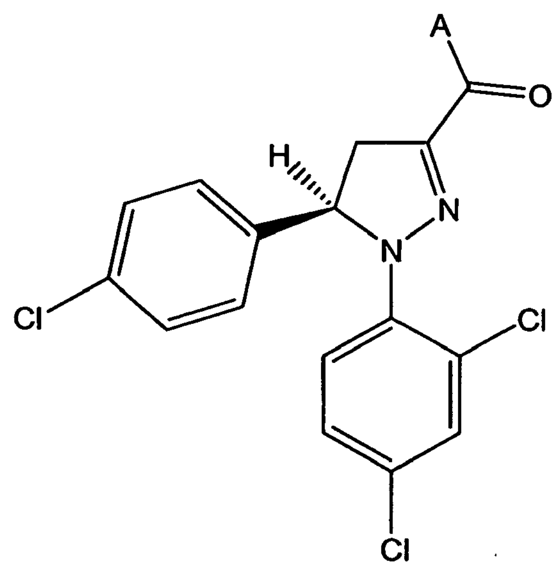

IIIa

, wherein A represents a leaving group, preferably a chlorine atom, said compound being optionally isolated and/or optionally purified, and one compound of formula (IIa) or general formula (IIIa) is reacted with a compound of formula VIII,

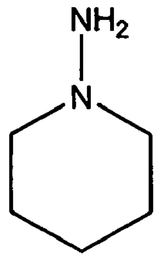

VIII

under inert atmosphere to yield (R)-N-piperidinyl-5-(4-chlorophenyl)-1-(2,4-dichlorophenyl)-4,5-dihydro-1H-pyrazole-3-carboxamide of formula (Ia),

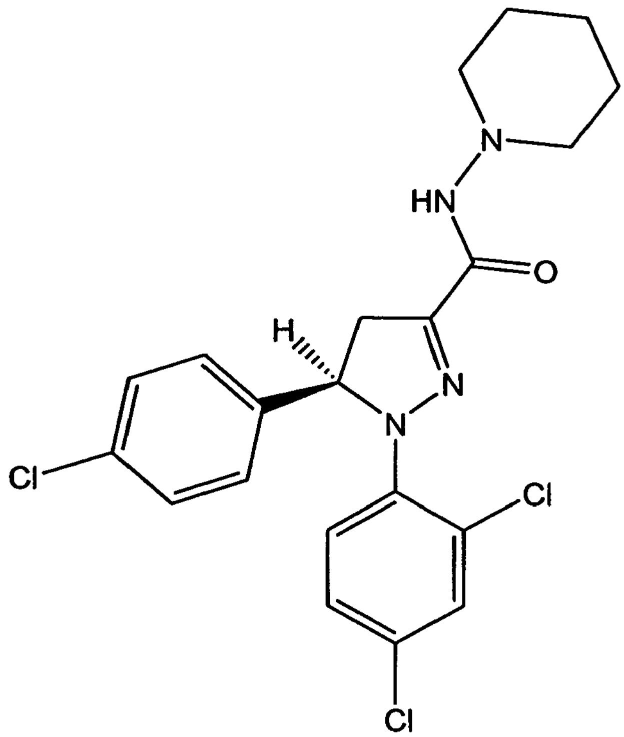

which is optionally isolated and/or optionally purified.

(S)-N-piperidinyl-5-(4-chlorophenyl)-1-(2,4-dichlorophenyl)-4,5-dihydro-1H-pyrazole-3-carboxamide may be prepared with a compound of formula IIb

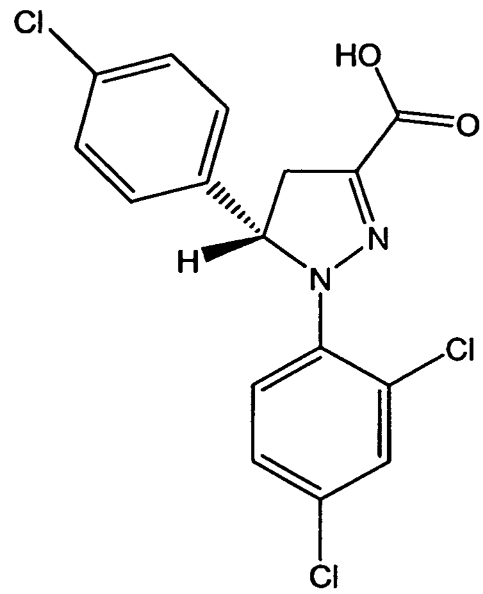

IIb

, which is optionally transferred under inert atmosphere to a compound of general formula (IIIb) via reaction with an activating agent

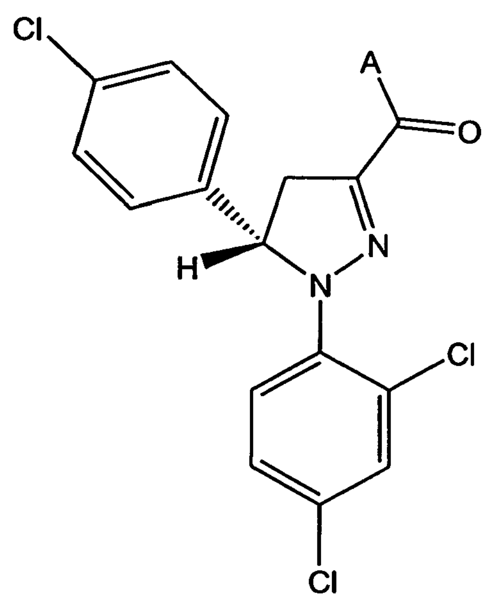

wherein A represents a leaving group, preferably a chlorine atom, said compound being optionally isolated and/or optionally purified, and one compound of formula (IIb) or general formula (IIIb) is reacted with a compound of formula VIII,

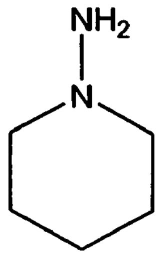

VIII

under inert atmosphere to yield (S)-N-piperidinyl-5-(4-chlorophenyl)-1-(2,4-dichlorophenyl)-4,5-dihydro-1H-pyrazole-3-carboxamide of formula (Ib),

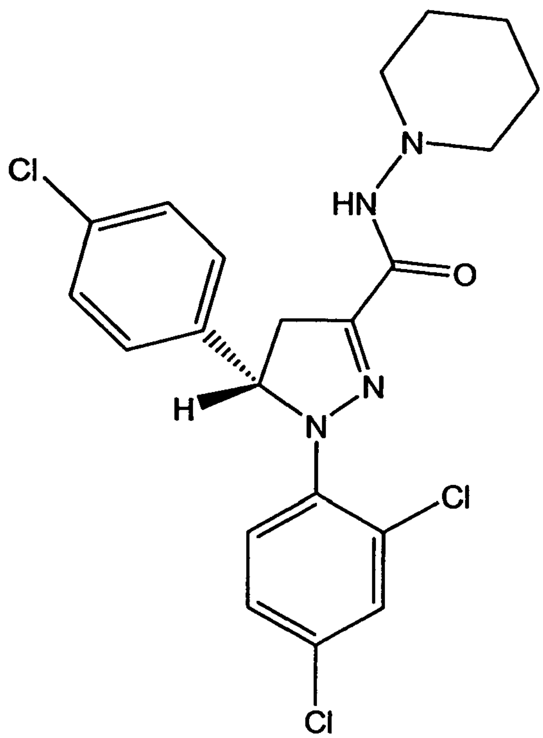

which is optionally isolated and/or optionally purified.

Preferably the compounds IIa or IIb are obtained from a mixture comprising the enantiomers

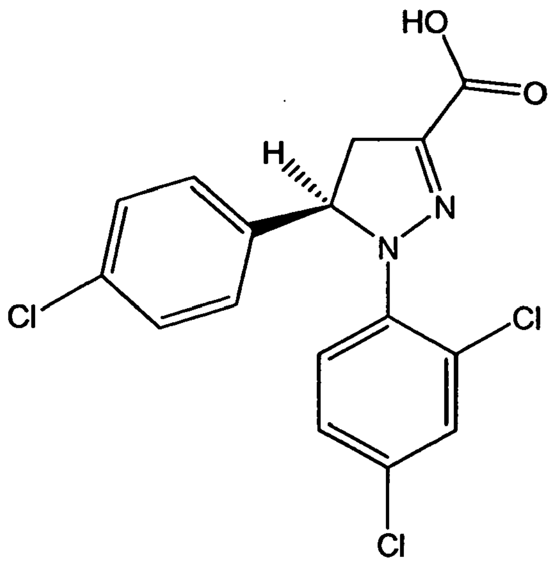

IIa and

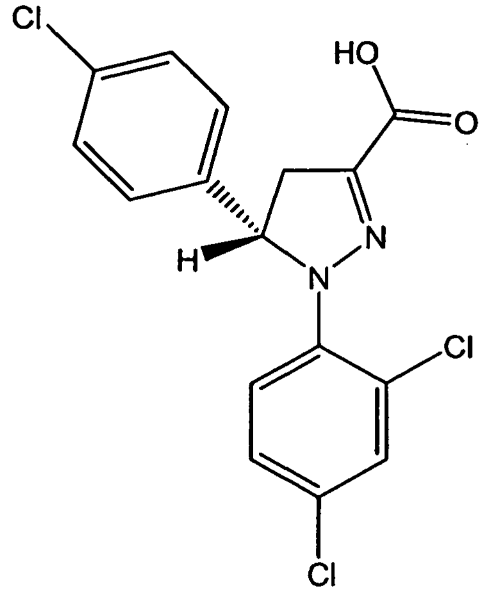

IIb

The compounds (IIa) or (IIb) may be obtained from the mixture of the enantiomers by means well known to those skilled in the art. Preferably, the compounds IIa or IIb are obtained from the mixture in form of an addition compound with a chiral base, preferably (+)-Cinchonine or R-(+)-1-Phenylethylamine, and preferably liberated from the addition compound.

Preferably the racemic mixture according to formula II

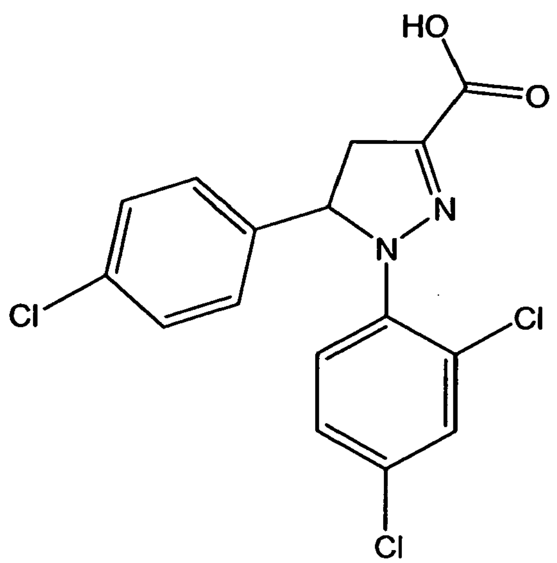

II

comprising the enantiomers IIa and IIb may be obtained by reacting one benzaldehyde compound of general formula IV

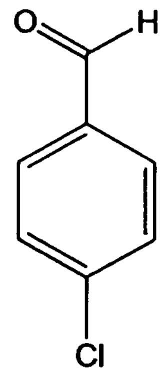

(IV)

with a pyruvate compound of formula (V)

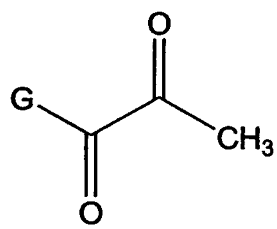

(V),

wherein G represents an OR group with R being a branched or unbranched $C_{1-6}$ alkyl radical or G represents an $O^-K$ group with K being a cation,
to yield a compound of formula (VI)

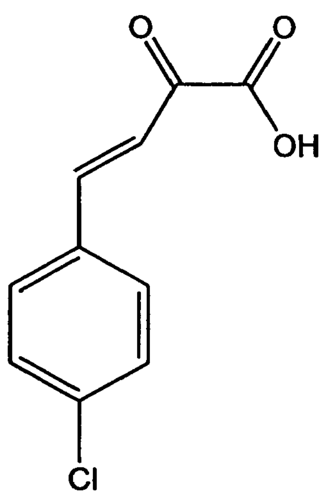

(VI)

which is optionally isolated and/or optionally purified, and which is reacted with an optionally substituted phenyl hydrazine of formula (VII)

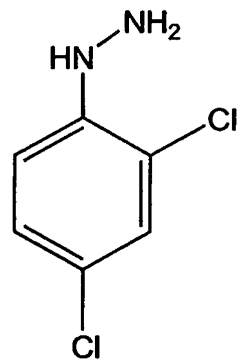

(VII)

or a corresponding salt thereof, under inert atmosphere, to yield a compound of formula (II)

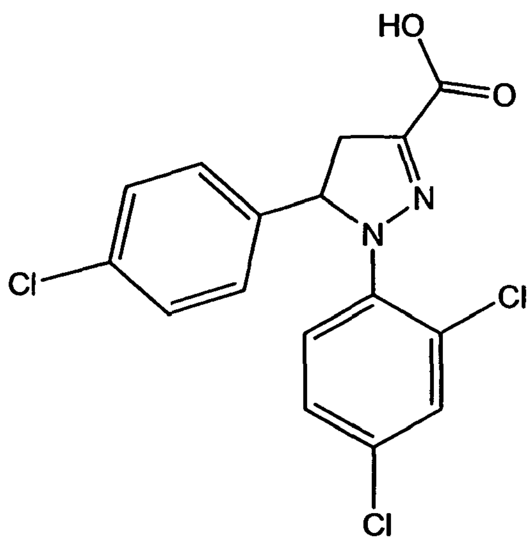

(II)

which is optionally isolated and/or optionally purified.

The production of the compound according to formula II is already described in detail in international patent applications WO2005/077909 and WO 2005/077911. The respective descriptions are hereby incorporated by reference and form part of the present disclosure.

The reactions of general formula (IIa) or (IIb) with a compound of formula VIII to yield compounds of general formula Ia or Ib, is preferably carried out in presence of a base such as triethylamine in a reaction medium such as methylenchloride. The temperature is preferably in the range from 0˚C to the boiling point of the reaction medium. The reaction time may vary over a broad range, e.g. from several hours to several days.

The aforementioned reaction involving the synthesis of the 4,5-dihydro-pyrazole ring preferably is carried out under an inert atmosphere, preferably nitrogen or argon, to avoid oxidation of the ring-system.

During the processes described above the protection of sensitive groups or of reagents may be necessary and/or desirable. The introduction of conventional protective groups as well as their removal may be performed by methods well-known to those skilled in the art.

The purification and isolation of (R)-N-piperidinyl-5-(4-chlorophenyl)-1-(2,4-dichlorophenyl)-4,5-dihydro-1H-pyrazole-3-carboxamide or (S)-N-piperidinyl-5-(4-chlorophenyl)-1-(2,4-dichlorophenyl)-4,5-dihydro-1H-pyrazole-3-carboxamide or any intermediate thereof may, if required, be carried out by conventional methods known to those skilled in the art, e.g. chromatographic methods or recrystallization.

The nonracemic mixture according to the invention is suitable as pharmaceutical active substance for the preparation of medicaments.

In particular it has been found that the components of the non-racemic mixture as well as the non-racemic mixture itself show little or no development of tolerance during treatment, particularly with respect to food intake, i.e: if the treatment is interrupted for a given period of time and then continued afterwards, the inventively used compound will again show the desired effect. After ending the treatment with the compound, the positive influence on the body weight is found to continue at least for a certain period of time.

Furthermore, this the components of the non-racemic mixture as well as the non-racemic mixture itself according to the invention is connected to relatively weak Herg channel affinity, thus a low risk of prolongation of the QT-interval is to be expected for this compound.

In summary, the inventively used non-racemic mixture according to the invention is distinguished by a broad spectrum of beneficial effects, while at the same time showing relatively little undesired effects, i.e. effects which do not positively contribute to or even interfere with the well being of the patient.

Thus, another aspect of the present invention relates to a medicament comprising the non-racemic mixture according to the invention, and optionally one or more pharmaceutically acceptable excipients.

Another aspect of the present invention is the use of the non-racemic mixture according to the invention, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Also preferred is the use of the non-racemic mixture according to the invention, and optionally one or more pharmaceutically acceptable excipients, for the prophylaxis and/or treatment of psychosis.

Also particularly preferred is the use of the non-racemic mixture according to the invention, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also very highly preferred is the use of the non-racemic mixture according to the invention, for the preparation of a medicament for the prophylaxis and/or treatment of food intake disorders, preferably bulimia, anorexia, cachexia, obesity, type II diabetis mellitus (non-insuline dependent diabetes mellitus), more preferably obesity, type II diabetis, metabolic syndrome and/or dyslipidaemia.

Also preferred is the use of the non-racemic mixture according to the invention, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also preferred is the use of the non-racemic mixture according to the invention, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of the non-racemic mixture according to the invention, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

Also particularly preferred is the use of the non-racemic mixture according to the invention, and optionally one or more pharmaceutically acceptable excipients, for the preparation of a medicament for the prophylaxis and/or treatment of one or more disorders selected from the group consisting of dementia and related disorders, preferably for the prophylaxis and/or treatment of one or more types of dementia selected from the group consisting of memory loss, vascular dementia, mild cognitive impairment, frontotemporal dementia and Pick's disease; binge eating disorders; juvenile obesity; drug induced obesity; atypical depression; behavioural addictions; attention deficit disorders; Tourette's syndrome; suppression of reward-related behaviours; e. g. conditioned place avoidance such as suppression of cocaine- and morphine induced conditioned place preference; impulsivity; sexual dysfunction; preferably for the prophylaxis and/or treatment of one or more types of sexual dysfunction selected from the group consisting of erectile difficulty and female sexual dysfunction; seizure disorders; nausea; emesis; neuroinflammatory disease, preferably for the prophylaxis and/or treatment of one or more types of neuroinflammatory diseases selected from the group consisting of multiple sclerosis, demyelinisation related disorders, Guillan-Barré syndrome, viral encephalitis and cerebrovascular accidents; neurological disorders; muscle spasticity; traumatic brain injury; spinal cord injury; inflammation and immunomodulatory disorders, preferably for the treatment and/or prophylaxis of one or more types of inflammation and immunomodulatory disorders selected from the group consisting of cutaneous T-cell lymphoma, rheumatoid arthritis, systemic lupus erythematosus, sepsis, sarcoidosis, idiopathic pulmonary fibrosis, bronchopulmonary dysplasia, retinal disease, scleroderma, renal ischemia, mycocardial infarction, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, Crohn's disease, inflammatory bowel disease, reversible airway obstruction, adult respiratory distress syndrome, chronic obstructive pulmonary disease and bronchitis; cerebral apoplexy; craniocerebral trauma; neuropathic pain disorders; gastric ulcers; atheriosclerosis and liver cirrhosis.

Dementia is a disease characterized by the progressive deterioration in cognitive and social adaptive functions that can eventually interfere with the patient's ability to live independently. Dementia also constitutes of impairment in short- and long-term memory plus additional symptoms, such as problems with abstract thinking, judgment, or personality. An estimated 18 million patients suffer from dementia worldwide. The most common forms of dementia include Alzheimer's disease and vascular dementia. Other forms are frontotemporal dementia and Pick's disease.

Dementia can also be of vascular origin. Vascular dementia (atherosclerotic cerebrovascular disease) is considered to be the second most common dementia of late life, affecting approximately 10-15% of all cases. Alzheimer's disease (AD) and vascular dementia can exist in isolation or together (mixed dementia). In vascular dementia, atherosclerotic changes in cerebral vessels can lead to reduced local blood flow that results in multiple small strokes (multi-infarct dementia). Vascular dementia is pharmacologically treated by stroke prophylaxis, and by treatment of the cognitive deficit.

Alzheimer's disease (AD), the most common and important form of dementia, is a neurodegenerative disorder that is characterized by progressive impairment of cognitive functions, such as abstract reasoning and memory. Currently, an estimated 2 million people in the United States and 12 million worldwide are afflicted by this disease. Due to increasing life expectancy, it is predicted that there will be over 100 million AD patients worldwide by the year 2050. AD is one of the most prevalent illnesses in the elderly. The majority of AD patients are in their sixties or older. More than 5% of all persons over the age of 70 have significant memory loss due to AD.

AD is mainly characterized through a gradual development of forgetfulness. In further advanced disease stages, other failures in cerebral function become increasingly apparent. This includes impairment of speech, writing, and arithmetic skills. Visiospacial orientation, such as parking the car, dressing properly, and giving and understanding directions to a location, can become defective or impaired. In late stage disease, patients forget how to use common objects and tools while retaining necessary motor power and co-ordination for these activities.

Schizophrenia is characterized by profound disruption in cognition and emotion, affecting the most fundamental human attributes: language, thought, perception, affect, and sense of self. Positive symptoms include psychotic manifestations, such as hearing internal voices or experiencing other sensations not connected to an obvious source (hallucinations) and assigning unusual significance or meaning to normal events or holding fixed false personal beliefs (delusions). Negative symptoms are characterized by affective flattening and lack of initiative or goals (avolition), loss of usual interests or pleasures (anhedonia), disturbances of sleep and eating, dysphoric mood (depressed, anxious, irritable, or angry mood) and difficulty concentrating or focusing attention.

Major depression is a multifaceted disorder characterized by primarily by dysphoric mood and loss of interest or pleasure in activities that were once enjoyable. Other physical and psychological symptoms include inability to concentrate, motor disturbances (psychomotor retardation or agitation), feelings of worthlessness, inappropriate guilt, thoughts of suicide, and disturbances in appetite and sleep.

Anxiety disorders are a group of syndromes that include generalized anxiety disorder, panic disorder, phobias, obsessive-compulsive disorder, and post traumatic stress disorder. Although each disorder has its own distinct features, all share common symptoms of excessive worrying, intense fears and dread, hypervigilance and/or somatic symptoms, in the absence of a dangerous situation.

Normal sexual function requires, among others, the ability to achieve and maintain penile erection. Major anatomic structures of the penis that are involved in erectile function include the corpus cavernosum, corpus spinosum, and the tunica albuginea (a collagenous sheath that surrounds each corpus). The corpora are composed of a mass of smooth muscle (trabecula) which contains a network of endothelial-lined vessels (lacunar spaces). Penile tumescence and erection is caused by relaxation of the arteries and corporal smooth muscles, while closing emissary veins, leading to increased blood flow into the lacunar network. Central and peripheral innervation contributes to regulation of the erectile response.

Erectile dysfunction (ED) may result from failure to initiate, fill, or store adequate blood volume within the lacunar network of the penis. Depending on the underlying dysfunction, ED may be vasculogenic, neurogenic, endocrinologic, diabetic, psychogenic, or medication-related.

ED affects 10-25% of middle-aged and elderly men, and has a profound impact on the well-being of affected men. It is currently treated using PDE5 inhibitors such as vardenafil, tadalifil, and sildenafil. Intraurethral alpostadil (prostaglandin EI) may be used in patients that fail on oral agents. In addition, vacuum constriction devices (VCD) are a well-established, noninvasive therapy.

Female sexual dysfunction (FSD) is highly prevalent, age-related, and progressive. It affects 30 to 50% of women. FSD denotes a range of medical problems and is categorized according to disorders of (1) desire, (2) arousal, (3) orgasm and (4) sexual pain, and symptoms include diminished vaginal lubrication, pain and discomfort with intercourse, decreased arousal, and difficulty achieveing orgasm. On a molecular level, vasoactive intestinal peptide (VIP), nitic oxide (NO), and sex hormones such as estrogens and androgens have been suggested to be important in female sexual function. Current treatment approaches include estrogen replacement therapy, methyl testosterone, PDE5 inhibitors such as sildenafil, the NO-donor L-arginine, prostaglandin El, phentolamine, and the dopamine agonists apomorphine.

Also preferred is the use of the non-racemic mixture according to the invention, and optionally one or more pharmaceutically acceptable excipients, for the preparation of a medicament for the prophylaxis and/or treatment of metabolic syndrome, especially weight independent, cardiovascular diseases, cardiovascular risk factors, glucose intolerance and insulin resistance; for the prophylaxis and/or treatment and/or influencing of dyslipidaemia; for the prophylaxis and/or treatment and/or influencing of blood parameters especially lipid parameters, seeming to lower LDL bodies while increasing HDL bodies, including also the treatment of food disorders (obesity) under conditions of developed diabetes, especially Type II.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.
The metabolic syndrome is characterized by an interaction of several physiological parameters such as triglycerides, lipids, blood pressure, glucose levels and insuline levels.

Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Since the non-racemic mixture according to the invention also has a beneficial effect on the ratio of low density lipoprotein (LDL) to high density lipoprotein (HDL), i.e. they lower the LDL-levels and/or elevate the HDL levels; they are also useful as coating material or co-coating material in stents in order to prevent restenosis.

The term "treatment" as used in the present application encompasses prevention, amelioration and/or complete recovery from the disease. Said term also includes the prevention, amelioration and/or complete recovery of one or more symptoms associated with the disease.

Furthermore, (R)-N-piperidinyl-5-(4-chlorophenyl)-1-(2,4-dichlorophenyl)-4,5-dihydro-1H-pyrazole-3-carboxamide according to the invention is connected to relatively weak Herg channel affinity, thus a low risk of prolongation of the QT-interval is to be expected for the compound.

In summary, the inventively used non-racemic mixture according to the invention is distinguished by a broad spectrum of beneficial effects, while at the same time showing relatively little undesired effects, i.e. effects which do not positively contribute to or even interfere with the well being of the patient.

Another aspect of the invention is the use of the non-racemic mixture according to the invention for the manufacture of a medicament for improvement of cardiovascular and/or metabolic risk factors, such as one or more of the following factors:

- Elevated triglycerides, whereby elevated levels of triglycerides are preferably understood as being > 150 mg/dl,
- Low HDL cholesterol, whereby low levels of HDL cholesterol are preferably understood as being < 40 mg/dl in men and < 50 mg/dl in women,
- Hypertension, whereby hypertension is preferably understood as being > 130/85 mmHg,
- Impaired fasting glucose, whereby impaired fasting glucose levels are preferably understood as being > 110 mg/dl,
- Insulin resistance
- Dyslipidemia.

Another aspect of the invention is the use of the non-racemic mixture according to the invention for the manufacture of a medicament for the treatment of the weight independent aspects of metabolic syndrome.
Another aspect of the invention is the use of the non-racemic mixture according to the invention for the manufacture of a medicament for the treatment of dyslipidemia.
Another aspect of the invention is a method for improving cardiovascular and/or metabolic risk factors, such as one or more of the following factors:

- Elevated triglycerides, whereby elevated levels of triglycerides are preferably understood as being > 150 mg/dl,
- Low HDL cholesterol, whereby low levels of HDL cholesterol are preferably understood as being < 40 mg/dl in men and < 50 mg/dl in women,
- Hypertension, whereby hypertension is preferably understood as being > 130/85 mmHg,
- Impaired fasting glucose, whereby impaired fasting glucose levels are preferably understood as being > 110 mg/dl,
- Insulin resistance
- Dyslipidemia,

in a subject, preferably a human.
Another preferred aspect of the invention is also a method of treatment encompassing all the abovementioned uses, wherein the non-racemic mixture according to the invention, is applied to a person in need thereof, treating metabolic syndrome, especially weight independent, cardiovascular diseases especially fighting cardiovascular risk factors, influencing the blood parameters, especially the lipid parameters, diabetes, especially type II, glucose intolerance and insulin resistance, bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit; alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction; cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer; food intake disorders, preferably bulimia, anorexia, cachexia, obesity and/or type II diabetus mellitus (non-insuline dependent diabetes mellitus), more preferably obesity; psychosis; disorders of the central nervous system, disorders of the immune system, disorders of the cardiovascular system, disorders of the endocrinous system, disorders of the respiratory system, disorders of the gastrointestinal tract or reproductive disorders.
The medicament according to the present invention may be in any form suitable for the application to humans and/or animals, preferably humans including infants, children and adults and can be produced by standard procedures known to those skilled in the art. The composition of the medicament may vary depending on the route of administration.
The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.
Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions,

emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams or also 20 to 200 mg of active substance to be administered during one or several intakes per day.

In another aspect the present invention also provides pharmaceutical formulation comprising

- the mixture according to the invention and (S)-N-piperidinyl-5-(4-chlorophenyl)-1-(2,4-dichlorophenyl)-4,5-dihydro-1H-pyrazole-3-carboxamide; or
- the mixture according to the invention and (R)-N-piperidinyl-5-(4-chlorophenyl)-1-(2,4-dichlorophenyl)-4,5-dihydro-1H-pyrazole-3-carboxamide; or
- the mixture according to the invention and (rac) -N-piperidinyl-5-(4-chlorophenyl)-1-(2,4-dichlorophenyl)-4,5-dihydro-1H-pyrazole-3-carboxamide; or
- (S)-N-piperidinyl-5-(4-chlorophenyl)-1-(2,4-dichlorophenyl)-4,5-dihydro-1H-pyrazole-3-carboxamide and (R)-N-piperidinyl-5-(4-chlorophenyl)-1-(2,4-dichlorophenyl)-4,5-dihydro-1H-pyrazole-3-carboxamide;

in separated compartments of the pharmaceutical formulation.

This aspect of the invention is based on the surprising findings and benefits of the non-racemic mixture according to the invention already described above the object of providing a solution tailored to the needs of an individual are addressed in a very similar manner. Possible forms, dosages, excipients etc. of the pharmaceutical formulation have already been explained above, e.g. for medicaments.

"Compartments", especially "separated compartments" according to the invention is defined as meaning parts of a pharmaceutical formulation which by their composition, or their position in the formulation can be identified as seperate entities, examples include layers on or in a pellet, layers on or in a tablet, different pellets, e.g. compressed to a tablet or filled into a capsule etc.

In a preferred embodiment of the pharmaceutical formulation according to the invention the separated compartments are layers or pellets.

In another aspect of the present invention also provides the use of (S)-N-piperidinyl-5-(4-chlorophenyl)-1-(2,4-dichlorophenyl)-4,5-dihydro-1H-pyrazole-3-carboxamide its respective salts, polymorphs or solvates, especially hydrates, for the preparation of a medicament for the prophylaxis and/or treatment of dyslipidaemia and other metabolic disturbances that result from the ecessive consumption of highly palatable macronutrient food sources.

This aspect is a major aspect of the current invention and is based on the surprising findings and benefits of (S)-N-piperidinyl-5-(4-chlorophenyl)-1-(2,4-dichlorophenyl)-4,5-dihydro-1H-pyrazole-3-carboxamide forming part of the non-racemic mixture according to the invention and besides the discussion/explanations already given above is discussed and explained in more detail in the experimental part below based on the direct experimental results.

In a preferred embodiment is the use according to the invention, wherein the other metabolic disturbances that result from the ecessive consumption of highly palatable macronutrient food sources are selected from obesity, central adiposity, insulin resistance/impaired glucose tolerance (pre-diabetes), Type 2 diabetes and Metabolic Syndrome; especially metabolic syndrome or diabetes type II.

The present invention is illustrated below with the aid of examples. These illustrations are given solely by way of example and do not limit the general spirit of the present invention.

**Examples:**

## CHEMICAL PART

**Abbreviations:**

| | |
|---|---|
| Eq. | Equivalent |

(continued)

| | |
|---|---|
| Proc. | Process |
| Solv. Cryst. | Solvent used in crystallisation experiment |
| T Cryst. | Temperature at which crystallisation experiment was carried out |
| 1st Cryst. | first crystallisatioN |
| 2nd Crys. | second crystallisation |
| r.t. | room temperature (≈ 20-25 ˚C) |

**General description:**

Racemic 5-(4-chlorophenyl)-1-(2,4-dichlorophenyl)-4,5-dihydro-pyrazole-3-carboxylic acid is optained as described in international patent applications WO2005/077909 (exemplified in example 0) and WO 2005/077911. The respective descriptions are hereby incorporated by reference and form part of the present disclosure.

The resolution of the racemate of N-piperidinyl-5-(4-chlorophenyl)-1-(2,4-dichlorophenyl)-4,5-dihydro-1H-pyrazole-3-carboxamide may be carried out by methods known to those skilled in the art, e.g. column chromatography leading to Compound A or Compound B as described below. The racemate of N-piperidinyl-5-(4-chlorophenyl)-1-(2,4-dichlorophenyl)-4,5-dihydro-1 H-pyrazole-3-carboxamide is obtained as described in international patent application WO 2005/077911. The respective description is hereby incorporated by reference and form part of the present disclosure.

However its intermediate, racemic 5-(4-chlorophenyl)-1-(2,4-dichlorophenyl)-4,5-dihydro-pyrazole-3-carboxylic acid may easily be separated into the respective enantiomers via reaction with a chiral base. The process for this resolution is described below.

**Resolution of the enantiomers of 5-(4-chlorophenyl)-1-(2,4-dichlorophenyl)-4,5-dihydro-pyrazole-3-carboxylic acid**

The resolution of the enantiomers of 5-(4-chlorophenyl)-1-(2,4-dichlorophenyl)-4,5-dihydro-pyrazole-3-carboxylic acid was carried out via reaction with the following chiral bases:

- Brucine
- Quinine
- (-)-Cinchonidine
- (+)-Cinchonine
- R-(+)-1-Phenylethylamine
- (1 R,2S)-(-)-Ephedrine hydrochloride
- (1S,2R)-(+)-Ephedrine hydrochloride.

In each case the reactions were carried out with 0.5 and 1 equivalents of base in respect to 1 equivalent of the acid compound and by using the following solvents

- Ethanol
- Acetone
- Acetonitril
- Dioxane
- Ethylacetate
- Chloroform.

The results are summarized in the following tables. It may be understood that the afore mentioned crystallisation experiments that are not reflected in the following tables did not yield crystals of the respective salts under the given conditions.

However, suitable conditions for crystallization of these salts can be determined by those skilled in the art via routine experiments.

In the following tables
Acid represents racemic 5-(4-chlorophenyl)-1-(2,4-dichlorophenyl)-4,5-dihydro-pyrazole-3-carboxylic acid
R-Acid represents the respective derivative of (R)-5-(4-chlorophenyl)-1-(2,4-dichlorophenyl)-4,5-dihydro-pyrazole-3-carboxylic acid
S-Acid represents the respective derivative of (S)-5-(4-chlorophenyl)-1-(2,4-dichlorophenyl)-4,5-dihydro-pyrazole-3-carboxylic acid

**Processes for crystallisation:**

**Process A:** A solution of the chiral base was added on top of a solution of the racemic acid at room temperature.
**Process C:** A solution of the racemic acid was added on top of a solution of the chiral base. The mixture was heated to reflux and solvent was added until dissolution was complete. The solution was left to crystallisation at r.t.
**Process D:** The chiral base was directly added on top of a solution of the racemic acid at room temperature.
**Process E:** The chiral base was directly added on top of a solution of the racemic acid at reflux temperature.
**Process F:** The solution of the salt was evaporated to dryness. The residue was dissolved in a minimum amount of the solvent under reflux heating. The solution was left to crystallisation at r.t.

**Resolution with Brucine**

| Brucine | | | | | | | | | |
|---|---|---|---|---|---|---|---|---|---|
| 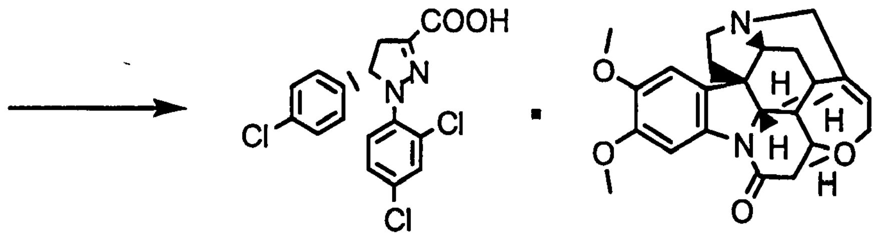 | | | | | | | | | |
| Acid g (mmol) | Eq. amine | Proc. | Solvent for crystallisation | T Cryst. | Yield 1st Cryst. % | % S-Acid | % R-Acid | Yield 2nd Cryst. % | % S-Acid |
| 0,4 g (1,09 mmol) | 0,5 | A | 8 ml acetone | r.t. | 44,6 | 98,8 | 1,2 | 5,6% | 99,4 |
| 0,4 g (1,09 mmol) | 0,5 | A | 11,5 ml acetonitrile | r.t. | 50,7 | 47,5 | 52,4 | | |
| 0,4 g (1,09 mmol) | 1 | A | 17 ml acetonitrile | r.t. | 25,6 | 45,7 | 54,3 | | |

**Resolution with Quinine**

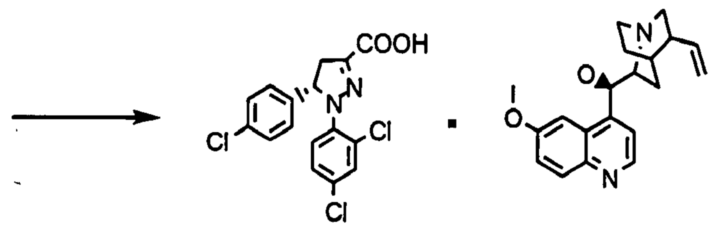

| Acid g (mmol) | Eq. amine | Proc. | Solvent for crystallisation | T Crys. | Yield 1st Cryst. % | % S-Acid | % R-Acid |
|---|---|---|---|---|---|---|---|
| 0,4 g (1,09 mmol) | 1 | A | 8 ml dioxane | r.t. | 49,46 | 91,6 | 8,3 |
| 0,4 g (1,09 mmol) | 1 | F | 15ml acetonitrile | r.t. | 26 | 94,4 | 5,5 |
| 0,4 g (1,09 mmol) | 1 | F | 2ml ethylacetate | r.t. | 25 | 96,7 | 3,3 |
| 0,4 g (1,09 mmol) | 0,5 | A | 4ml dioxane | r.t. | 38,5 | 97,5 | 2,5 |

**Resolution with (-)-Cinchonidine**

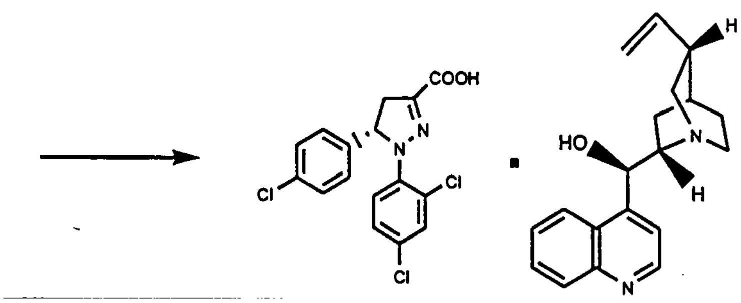

| Acid g (mmol) | Eq. amine | Proc. | Solvent for crystallisation | T Cryst. | Yield 1st Cryst. % | % S-Acid | % R-Acid |
|---|---|---|---|---|---|---|---|
| 0,4 g (1,09 mmol) | 1 | F | 2ml dioxane | r.t. | 31 | 94,4 | 5,6 |
| 0,4 g (1,09 mmol) | 1 | F | 19ml Ethylacetate | r.t. | 28,5 | 95,8 | 4,2 |
| 0,4 g (1,09 mmol) | 1 | F | 20ml acetone | r.t. | 19,6 | 96,9 | 3,1 |
| 0,4 g (1,09 mmol) | 1 | F | 24ml acetonitrile | r.t. | 42 | 85,8 | 14,1 |

**Resolution with (+)-Cinchonine**

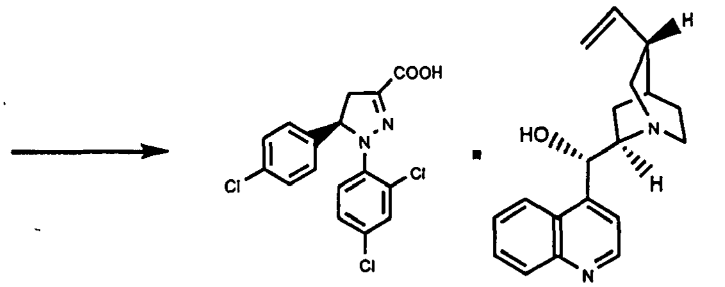

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | C | 50 ml acetone | r.t. | 32,8 | 7,42 | 92,57 |
| 0,4 (1,09 mmol) | 0,5 | F | 30ml acetone | r.t. | 23,5 | 4,0 | 95,9 |
| 0,4 (1,09 mmol) | 0,5 | C | 50 ml acetonitrile | r.t. | 31,5 | 3,07 | 96,92 |
| 0,4 (1,09 mmol) | 1 | C | 50 ml acetonitrile | r.t. | 78,25 | 39,01 | 60,98 |
| 0,4 (1,09 mmol) | 0,5 | C | 15 ml ethylacetate | r.t. | 14,9 | 3,62 | 96,37 |
| 0,4 (1,09 mmol) | 1 | C | 27 ml ethylacetate | r.t. | 35 | 7,78 | 92,21 |
| 0,4 (1,09 mmol) | 1 | F | 4ml dioxane | r.t. | 21 | 33,5 | 66,4 |

**Resolution with R-(+)-1-Phenylethylamine**

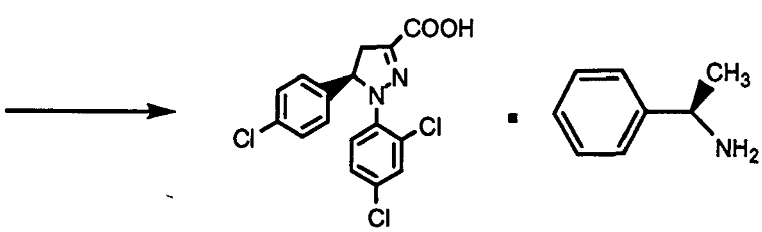

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | D | 1,6ml ethanol | r.t. | 22 | 51,4 | 48,6 |
| 0,4 (1,09 mmol) | 0,5 | E | 6ml acetonitrile | r.t. | 11 | 8,1 | 91,9 |
| 0,4 (1,09 mmol) | 1 | E | 6ml acetonitrile | r.t. | 50 | 36,8 | 63,2 |

(continued)

| Acid | Eq. amine | Proc. | Solvent for crystallisation | T Cryst. | Yield 1st Cryst. % | % S-Acid | % R-Acid |
|---|---|---|---|---|---|---|---|
| 0,4 (1,09 mmol) | 1 | E | 6ml dioxane | ≈5˚C | 5 | 4,6 | 95,3 |

**Detailed Description:**

**<u>Chemical Example 1: (R)-N-piperidinyl-5-(4-chlorophenyl)-1-(2,4-dichlorophenyl)-4.5-dihydro-1H-pyrazole-3-carboxamide (Compound A)</u>**

**Resolution of 5-(4-chlorophenyl)-1-(2,4-dichlorophenyl)-4,5-dihydro-pyrazole-3-carboxylic acid with (+)-Cinchonine**

35 g (294.68 mmol) of racemic 5-(4-chlorophenyl)-1-(2,4-dichlorophenyl)-4,5-dihydro-pyrazole-3-carboxylic acid in 630 m acetonitril were added to a suspension of 16.39 g (47.34 mmol) of (+)-Cinchonine in 920 ml acetonitrile under vigorous stirring. The resulting suspension was heated to reflux and acetonitrile (1300 ml) was added until dissolution was complete. The resulting solution was left to crystallise at room temperature over the weekend, whereupon a crystalline solid was obtained. The crystalline solid was filtered off, washed with 50 ml of cold acetonitrile and dried to give 25.26 g of a white solid (ee ≈ 91-92%). No second fraction of crystals could be obtained from the mother liquors, neither upon cooling in the refrigerator nor upon concentration. Thus, recrystallization of the diastereomeric salt was carried out in different solvents in order to improve the enantiomeric excess:

| Solvent | Yield | Ratio of enantiomers % (R) / % (S) |
|---|---|---|
| Acetonitrile | 90 % | 99,6 / 0,4 |
| EtOH-$H_2O$ | 68,5 % | 99,3/0,7 |
| Isopropanol | 55 % | 98,9/1,1 |

Consequently, the product was recrystallized from acetonitrile to give 22.6 g of crystals (yield 72 mol-% related to chiral base). The ratio of enantiomers determined by capillary electrophoresis was:

99.7 % of (R)-5-(4-chlorophenyl)-1-(2,4-dichlorophenyl)-4,5-dihydro-pyrazole-3-carboxylic acid (+)-Cinchonine
0.3 % of (S)-5-(4-chlorophenyl)-1-(2,4-dichlorophenyl)-4,5-dihydro-pyrazole-3-carboxylic acid (+)-Cinchonine ee= 99.4 %.

**Preparation of (R)-5-(4-chlorophenyl)-1-(2,4-dichlorophenyl)-4,5-dihydro-pyrazole-3-carboxylic acid from addition compound (R)-5-(4-chlorophenyl)-1-(2,4-dichlorophenyl)-4,5-dihydro-pyrazole-3-carboxylic acid-(+)-Cinchonine**

22.59 g (34 mmol) of the diastereomeric salt, which was recrystallized from acetonitrile, were suspended in 200 ml of 6N HCl and stirred at room temperature for 15 minutes. Afterwards, 200 ml of toluene were added until dissolution was complete. The mixture was stirred for 30 minutes and the phases were separated. The aqueous phase was extracted with toluene, the combined organic phases were washed with water, dried over sodium sulfate, filtered and evaporated to dryness, whereupon 11.9 g (95 %) of a white microcrystalline solid (melting point 129-133) were obtained.

Enantiomeric excess determined bY capillary electrophoresis. ee = 99.2 %
Chemical purity determined by HPLC: 99,3 %
$[\alpha]_D$ (c=1,23˚C, MeOH) = -429.

**Isolation of the enantiomer (R)-5-(4-chlorophenyl)-1-(2,4-dichlorophenyl)-4,5-dihydro-pyrazole-3-carboxylic acid from the mother liquors obtained from the crystallisation of the enantiomer (S)-5-(4-chlorophenyl)-1-(2,4-dichlorophenyl)-4,5-dihydro-pyrazole-3-carboxylic acid with Brucine**

The residue obtained from the evaporation of the mother liquors obtained from the crystallisation of (S)-5-(4-chlorophenyl)-1-(2,4-dichlorophenyl)-4,5-dihydro-pyrazole-3-carboxylic acid with Brucine (see below) was treated with

6N HCl as described above for obtaining the free acid. After evaporating the organic phase 21.86 g of a mixture with a theoretical composition of 20 mmoles (S)-5-(4-chlorophenyl)-1-(2,4-dichlorophenyl)-4,5-dihydro-pyrazole-3-carboxylic acid and 39.9 mmoles of (R)-5-(4-chlorophenyl)-1-(2,4-dichlorophenyl)-4,5-dihydro-pyrazole-3-carboxylic acid were obtained. A suspension of 13.82 g (39.9 mmoles) of (+)-cinchonine in 1000 ml of acetonitrile were added and the mixture was heated to reflux. More acetonitrile was added until dissolution was complete (total volume of acetonitrile 4000 ml). The mixture was then slowly cooled to room temperature to obtain crystals. After a few hours a white solid was obtained, which was filtered off and dried to give 22.42 (yield 84,5% related to the salt of (R)-5-(4-chlorophenyl)-1-(2,4-dichlorophenyl)-4,5-dihydro-pyrazole-3-carboxylic acid).

Enantiomeric excess as determined by capillary electrophoresis was ee = 92 %.

The purity of the isolated salt was comparable to the one of the salt obtained directly from the resolution of the racemate. Consequently, the same purification process as described above could be applied.

**Resolution of racemic 5-(4-chlorophenyl)-1-(2,4-dichlorophenyl)-4,5-dihydro-pyrazole-3-carboxylic acid with Brucine**

Preparation of the diastereomeric salt with Brucine

At room temperature 15.74 g (39.9 mmol) of Brucine were dissolved in 475 ml of acetone under vigorous stirring. A solution of 29.5 g (79.8 mmol) of the racemic 5-(4-chlorophenyl)-1-(2,4-dichlorophenyl)-4,5-dihydro-pyrazole-3-carboxylic acid in 85 ml acetone was added. The resulting mixture was allowed to crystallise for a couple of days, whereupon a crystalline solid was obtained. The crystalline solid was filtered off, washed with cold acetone and dried to yield 13.66 g (45 mol % related to the base) of a beige-coloured solid were obtained.

The ratio of the enantiomers as determined by capilar electrophoresis was:

99 % of (S)-5-(4-chlorophenyl)-1-(2,4-dichlorophenyl)-4,5-dihydro-pyrazole-3-carboxylic acid Brucine

1 % of (R)-5-(4-chlorophenyl)-1-(2,4-dichlorophenyl)-4,5-dihydro-pyrazole-3-carboxylic acid Brucine

Enantiomeric excess ee = 98 %

The filtered mother liquors were left in the refrigerator and yielded a second fraction of crystals, which were filtered off and dried to give another 1.56 g (5.1 molar % related to the base).

The analysis of the 2nd fraction via capillar electrophoresis gave
99.3 % of (S)-5-(4-chlorophenyl)-1-(2,4-dichlorophenyl)-4,5-dihydro-pyrazole-3-carboxylic acid Brucine
0.7 % of (R)-5-(4-chlorophenyl)-1-(2,4-dichlorophenyl)-4,5-dihydro-pyrazole-3-carboxylic acid Brucine
Enantiomeric excess ee = 98.6 %

Total yield:

15.22 g (50.1 mol-% related to base) of the salt with ee = 98 %.

**Determination of the chirality of the Brucine salt via X-ray crystallography**

The chirality of the enantiomer that crystallized with Brucine (ee 98 %) was determined via x-ray crystallography and corresponds to the S-enantiomer. Accordingly, the other enantiomer that crystallized with (+)-cinchonine is the corresponding R-enantiomer.

**Preparation of (S)-5-(4-chlorophenyl)-1-(2,4-dichlorophenyl)-4,5-dihydro-pyrazole-3-carboxylic acid from addition compound (S)-5-(4-chlorophenyl)-1-(2,4-dichlorophenyl)-4,5-dihydro-pyrazole-3-carboxylic acid brucine**

15.17 g (19.85 mmol) of (S)-5-(4-chlorophenyl)-1-(2,4-dichlorophenyl)-4,5-dihydro-pyrazole-3-carboxylic acid Brucine were suspended in 150 ml of 6N HCl and the suspension was stirred for 10 minutes, followed by the addition of 150 ml of toluene upon which complete dissolution was observed. The resulting mixture was stirred for an additional 30 minutes under control with column chromatography (silical gel, $Cl_2CH_2$:MeOH 95:5). Afterwards, the phases were separated, the aqueous phase was extracted with toluene and the combined toluene phases were washed with water three times, dried over sodium sulfate, filtered and evaporated to dryness. 7 g (95,5 %) of an oil were obtained, which solidified upon addition of a small amount of diethylether to yield a white amorphous solid that showed a crystalline transformation under melting at 130-133 ˚C.

CCF($Cl_2CH_2$:MeOH 95:5) : $R_f$ = 0.3
The analysis of the enantiomers via capillar electrophoresis gave
99.1 % (S)-5-(4-chlorophenyl)-1-(2,4-dichlorophenyl)-4,5-dihydro-pyrazole-3-carboxylic acid
0.9 % (R)-5-(4-chlorophenyl)-1-(2,4-dichlorophenyl)-4,5-dihydro-pyrazole-3-carboxylic acid
Enantiomeric excess ee = 98.2 %
[□]$_D$ (c=1,23˚C, MeOH) = -480.5.
Chemical purity determined by HPLC: 99.1 %

$^1$H-NMR ($CDCl_3$) δ ppm: 3,2 (dd, J=6,4 y 18,2 Hz, 1H), 3,7 (dd, J=12,7 y 18,2 Hz, 1 H), 5,85 (dd, J=6,4 y 12,6 Hz, 1 H), 7,0-7,2 (m, 7H).

**Isolation of the enantiomer (S)-5-(4-chlorophenyl)-1-(2,4-dichlorophenyl)-4,5-dihydro-pyrazole-3-carboxylic acid from the mother liquors obtained from the crystallisation of the other enantiomer (R)-5-(4-chlorophenyl)-1-(2,4-dichlorophenyl)-4,5-dihydro-pyrazole-3-carboxylic acid with (+)-cinchonine**

The residue obtained from the evaporation of the mother liquors obtained from the crystallisation of (R)-5-(4-chlorophenyl)-1-(2,4-dichlorophenyl)-4,5-dihydro-pyrazole-3-carboxylic acid with (+)-Cinchonine was treated with 6N HCl as described above for obtaining the free acid. After evaporating the organic phase 23.63 g of a mixture with a theoretical composition of 13.34 mmoles of (R)-5-(4-chlorophenyl)-1-(2,4-dichlorophenyl)-4,5-dihydro-pyrazole-3-carboxylic acid and 47.34 mmoles of (S)-5-(4-chlorophenyl)-1-(2,4-dichlorophenyl)-4,5-dihydro-pyrazole-3-carboxylic acid were obtained. Said mixture, dissolved in 118 ml of acetone, was added on top of a solution of 18.7 g (47.4 mmoles) of brucine in 650 ml of acetone, stirred for a couple of minutes and left to crystallise at room temperature overnight. A creme-coloured solid was obtained that was filtered off and dried to yield 28 g (77.5 % relative to the (S)-salt).

Enantiomeric excess as determined by capilar electrophoresis was ee = 98.2 %

The purity of the isolated salt was comparable to the one of the salt obtained directly from the resolution of the racemate. Consequently, the same purification process as described above could be applied.

**Preparation of (R)-N-piperidinyl-5-(4-chlorophenyl)-1-(2,4-dichlorophenyl)-4.,5-dihydro-1H-pyrazole-3-carboxamide (Compound A)**

9.94 g (26.89 mmol) of (R)-5-(4-chlorophenyl)-1-(2,4-dichlorophenyl)-4,5-dihydro-pyrazole-3-carboxylic acid were dissolved in 95 ml of toluene, followed by the addition of 2.35 ml (32.3 mmoles) of thionyl chloride and 4 drops of DMF. Under anhydrous nitrogen atmosphere the mixture was heated to a temperature of 75-80˚ C under vigorous stirring for two hours and then allowed to cool to room temperature. Said acid chloride solution was then added dropwise to a solution of 3.47 ml (31.2 mmol) of N-Aminopiperidine, 15 ml (107.6 mmol) of triethylamine and 38 ml of anhydrous toluene, which was cooled to 0-5 ˚C, thereby keeping the temperature below 10 ˚C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium sulfate, filtered off and evaporated to dryness to yield 13.41 g of light yellow oil, which failed to crystallise. The oil was purified via column chromatography over silica gel (eluent: petrol ether ethylacetate 90:10 to 60: 40) to give 10.5 g (83.5 %) of (R)-N-piperidinyl-5-(4-chlorophenyl)-1-(2,4-dichlorophenyl)-4,5-dihydro-1H-pyrazole-3-carboxamide in form of an amorphous solid of light yellow colour (melting point 75-78 ˚C).

$^1$H-NMR (DMSO-$d_6$) δ ppm : 9,25 (s, 1H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1Hz, 1H), 3,0 (dd, J=5,7 and 18,1Hz, 1H), 2,75 (m, 4H), 1,5 (m, 4H), 1,2 (m, 2H).

Analysis of the enantiomer via chiral HPLC: ee = 100 %
Chemical purity determined by HPLC: 98,5%
$[\alpha]_D$ (c=1, 23˚C, MeOH) = - 293,5

**Preparation of Hydrochloride salt of (R)-N-piperidinyl-5-(4-chlorophenyl)-1-(2,4-dichlorophenyl-4,5-dihydro-1H-pyrazole-3-carboxamide (Compound A-Hydrochloride)**

0.5 g (1.1 mmol) of (R)-N-piperidinyl-5-(4-chlorophenyl)-1-(2,4-dichlorophenyl)-4,5-dihydro-1 H-pyrazole-3-carboxamide were dissolved in 10 ml isopropanol, cooled with ice andistsaturated HCl-ethanol solution was added. The solution changed its colour and a white solid formed, which was filtered off, washed with isopropanol:diethylether (1:1) and dried to yield 0.38 g (71 %) of a white solid with a melting point of 160-165 ˚C. The purity determined by HPLC was 99.3 %. Determination of chlorine: 7.15 % (98.5 % of the theoretical value).

$^1$H-NMR (DMSO-$d_6$) δ ppm : 10,3 (bs, 1H), 7,5 (m, 2H), 7,3 (2d, J= 2,5 y 8,5Hz, 3H), 7,15 (d, J=8,5Hz, 2H),

5,8 (dd, J=6,3 y 12,0Hz, 1H), 3,7 (dd+$H_2O$), 3,05 (m, 5H), 1,7 (m, 4H), 1,4 (m, 2H).

**Chemical Example 2:**

**Preparation of (S)-N-piperidinyl-5-(4-chlorophenyl)-1-(2,4-dichlorophenyl)-4,5-dihydro-1H-pyrazole-3-carboxamide (Compound B)**

5.27 g (14.26 mmol) of (S)-5-(4-chlorophenyl)-1-(2,4-dichlorophenyl)-4,5-dihydro-pyrazole-3-carboxylic acid were dissolved in 30 ml of toluene, followed by the rapid dropwise addition of 1.25 ml (17.11 mmoles) of thionyl chloride and 7 drops of DMF.Under anhydrous nitrogen atmosphere the mixture was heated to a temperature of 75-80° C under vigorous stirring for two hours and then allowed to cool to room temperature. Said acid chloride solution was then added dropwise to a solution of 1.84 ml (16.54 mmol) of N-Aminopiperidine, 8 ml (57 mmol) of triethylamine and 25 ml of anhydrous toluene, which was cooled to 0-5 °C, thereby keeping the temperature below 10 °C during the addition. The reaction mixture was stirred at room temperature overnight, whereupon a suspension was obtained. Water and Toluene were added to said suspension until dissolution was complete and the phases were separated. The aqueous phase was extracted with toluene, the combined organic phases washed with an NaOH solution (10 %) and water, dried over sodium sulfate, filtered off and evaporated to dryness to yield 6.85 g of an oil, which failed to crystallize using the solvents Ethanol, Ethylacetate and Acetone. The oil was purified via column chromatography over silica gel (eluent: petrol ether ethylacetate 90: 10 to 50: 50) to give 4.7 g (73 %) of (S)-N-piperidinyl-5-(4-chlorophenyl)-1-(2,4-dichlorophenyl)-4,5-dihydro-1H-pyrazole-3-carboxamide in form of an amorphous solid of light yellow color (melting point 73-76 °C).

$^1$H-NMR (DMSO-$d_6$) $\delta$ ppm : 9,25 (s, 1 H), 7,5 (d, J=8,8Hz, 1H), 7,4 (d, J=2,3Hz, 1H), 7,3-7,25 (2d, J=8,8 and 8,5Hz, 3H), 7,1 (d, J=8,5Hz, 2H), 5,8 (dd, J=5,7 and 11,8 Hz, 1H), 3,65 (dd, J=11,8 and 18,1Hz, 1H), 3,0 (dd, J=5,7 and 18,1Hz, 1H), 2,75 (m, 4H), 1,5 (m, 4H), 1,2 (m, 2H).

Analysis of the enantiomer via chiral HPLC: ee = 98 %
Chemical purity determined by HPLC: 98,7%

**Preparation of Hydrochloride salt of (S)-N-piperidinyl-5-(4-chlorophenyl)-1-(2,4-dichlorophenyl)-4,5-dihydro-1H-pyrazole-3-carboxamide (Compound B-Hydrochloride)**

0.2 g (0.4 mmol) of (S')-N-piperidinyl-5-(4-chlorophenyl)-1-(2,4-dichlorophenyl)-4,5-dihydro-1H-pyrazole-3-carboxamide were dissolved in 3 ml isopropanol, cooled with ice and a saturated HCl-ethanol solution was added. The solution changed its color and a white solid formed, which was filtered off, washed with isopropanol:diethylether (1:1) and dried to yield 0.14 g (72 %) of a white solid with a melting point of 165-175 °C. The purity determined by HPLC was 99.3 %. Determination of chlorine: 7.15 % (98.5 % of the theoretical value).

$^1$H-NMR (DMSO-$d_6$) $\delta$ ppm : 10,4 (bs, 1 H), 7,45 (m, 2H), 7,3 (2d, J= 2,6 y 8,5Hz, 3H), 7,15 (d, J=8,5Hz, 2H), 5,8 (dd, J=6,3 y 12,0Hz, 1H), 3,7 (dd, J=12,0 y 18,0Hz, 1H), 3,05 (m, 5H), 1,7 (m, 4H), 1,4 (m, 2H).

**PHARMACOLOGICAL PART**

**Example 1 Determination of the affinity of COMPOUND A and COMPOUND B for rat CB1 receptors.**

**Methods**

Binding affinity to CB1 receptor was evaluated according to the method of Govaerts et al (2004) with the following modifications. Briefly, cerebella from male Wistar rats (250-300g) were carefully dissected on ice and homogenates were prepared in cold 50 mM Tris-HCl, 5 mM $MgCl_2$, 1 mM EDTA, 0.25 M sucrose, pH 7.4 with a Potter-Heljheim homogeniser and the suspension was centrifuged at 1,000 x g for 5 minutes. The supernatants were collected and centrifuged at 50,000 x g for 15 min. The resulting pellets were resuspended in Tris-HCl buffer without sucrose, homogenized and incubated for 15 min at 37°C in an orbital shaker bath and centrifuged again at 50,000 x g for 15 min. Pellets were weighed, resuspended in Tris-HCl buffer without sucrose, homogenized with an Ultraturrax homogeniser at 13,500 rpm for 3 x 5 seconds and aliquoted in 0.9 ml volumes in Eppendorf tubes. Aliquots were centrifuged at 20,800 x g for 5 minutes, supernatants discarded and pellets were frozen at -80°C until use. Total protein concentration was determined using a Bio-Rad commercial assay base on the Lowry method. Competitive binding experiments were performed in presence of 1 nM [$^3$H]-CP 55,940 in siliconized glass tubes containing 100 $\mu$g protein/tube resuspended in 1 ml final volume of 50 mM Tris-HCl, 5 mM $MgCl_2$, 1 mM EDTA, 0.5% (w/v) bovine serum albumin, pH 7.4. Competitors were present at various concentrations and the non-specific binding was determined in the presence of 10 $\mu$M HU-210. After 1 hour incubation at 30°C, the suspension was rapidly filtered through 0.5% PEI pre-treated GF/B fiber filters on a 96-well harvester and washed 3 times with 3ml ice-cold binding buffer without bovine serum albumin. Radioactivity on filters

was measured with Wallac Winspectral 1414 counter by liquid scintillation in 6 ml Ecoscint H (National Diagnostics, U.K.). Assays were made in triplicate.

Binding data were analyzed by non-linear regression with the software GraphPad Prism Version 3.03.

### Results

When the affinity of COMPOUND A and COMPOUND B for the rat CB1 receptor subtype was determined in vitro, COMPOUND A was observed to have good to moderate affinity for these receptors with an $IC_{50}$ of 22 nM (16.1 nM in another measurement) (Table 1). On the other hand, with an $IC_{50}$ value of 813 nM, COMPOUND B has no pharmacologically relevant affinity for rat CB1 receptors (Table 1).

Table 1 The affinities of various compounds for rat CB1 receptors

| Example | $pIC_{50}$ | $IC_{50}$ |
|---|---|---|
| Compound B | 6.09 ±0.09 | 813 |
| Compound A | 7.66 ± 0.08 | 22 |
| | | 16.1 |

### Example 2: Effects of COMPOUND A and COMPOUND B on food intake, body weight, adiposity, dyslipidaemia and insulin resistance in rats that have become obese through the excessive consumption of highly palatable foods sources

### Animals

The experiment was performed on female Wistar rats (originally in the weight range 250-300 g). The rats were housed in pairs in polypropylene cages with solid floors and sawdust bedding at a temperature of 21±4°C and 55±20% humidity. Animals were maintained on a reverse phase light-dark cycle (lights off for 8 h from 09.30-17.30 h) during which time the room was illuminated by red light. Animals had free access to powdered high fat diet (VRF1 plus 20% lard), ground chocolate, ground peanuts and tap water at all times. The three different diets were contained in separate glass feeding jars with aluminum lids with a 3-4 cm hole cut in it to allow access to the food. Animals were housed in pairs for twelve weeks for the induction of obesity. Animals were then housed singly in polypropylene cages with wire grid floors to enable the food intake of each rat to be recorded. Polypropylene trays with cage pads were placed beneath each cage to detect any food spillage. Animals were accustomed to these new conditions (individual housing and the wire-grid floor cages) for at least 14 days before the start of the baseline readings.

### Experimental procedures for the feeding study

Approximately a week before the start of the study, animals were weighed (to the nearest 0.1 g using an electronic top-pan balance) and allocated into 3 weight-matched treatment groups, each containing 9-10 animals. Following a 7 day baseline run-in period, during which the animals were dosed by the appropriate route (eg orally) once a day with vehicle (0.5% hydroxy-propyl-methylcellulose [HPMC]; 1 ml/kg) or the test drug, ie COMPOUND A (10 and 30 mg/kg po) and COMPOUND B (10 and 30 mg/kg po) once daily for 28 days.

Food intake and body weight were measured once daily. Variations in body weight and energy levels of the different types of food were accounted for by expressing the food intake results in terms of kJ/kg rat weight.

Statistical comparisons between the body weights and daily food intakes of the different treatment groups have been made by analysis of covariance followed by Dunnett's test to compare each treatment group with the vehicle-treated controls. For the body weight data (body weights each day), the weights of the animals on Day 1 (ie immediately before the first drug treatment) were used as the covariate. Thus, means were adjusted for differences in body weight between the groups on Day 1 and standard errors of the mean (SEM) were calculated from the residuals of the statistical model. All mean body weight values on Day 1 are identical (and SEM are therefore zero) because the analysis of covariance adjusts them all to equal the 'grand mean' of the values of this day.

### Determination of carcass fat content

Total ether-extractable fat in the freeze-dried samples was estimated by the Soxhlet principle using a Foss Soxtec HT2 system. The protocol employed was based on the manufacturer's recommendations for meat samples, although freeze-dried samples obviated the necessity for either a fat hydrolysis step or mixing the sample with sand to

ensure efficient extraction.

Samples of approximately 1 g were accurately weighed into 26 mm x 60 mm cellulose extraction thimbles which were then plugged with approximately 0.8 g cotton wool. Sample fat was extracted into pre-weighed (including a few boiling chips) standard metal extraction cups using 70 ml of petroleum ether at 40-60˚C, a circulator temperature of 115˚C and a cooling flow rate of 2 l/min. A boiling stage of 25 min was employed, followed by a 40 min wash stage. A 10 min drying stage sufficed to remove ether from the extraction cups.

Percentage fat in the freeze-dried sample was calculated as follows:

$$\% \text{ fat} = [100 \times (\text{final cup weight} - \text{initial cup weight})]/\text{sample weight}$$

Percentage fat in the original sample was calculated using the % water as follows:

$$\% \text{ fat in original sample} = \% \text{ fat} \times [(100 - \% \text{ water})/100]$$

**Determination of carcass protein content**

Total protein in the freeze-dried samples was estimated using the Kjeldahl principle. A micro-Kjeldahl procedure was performed using a Foss 2012 digestion block and a Foss 2200 distilling unit. The protocol used was based on the manufacturer's recommendations for meat samples.

Approximately 0.3 g of freeze-dried sample was accurately weighed into 100 ml digestion tubes (Foss UK Ltd) in racks of 12 tubes. Each rack included at least one blank tube to determine a reagent blank. To each tube was added two Kjeltabs CQ and one Antifoam S tablet followed by 10 ml of 98% sulphuric acid. Samples were digested at 420˚C for 1 h to convert protein into ammonium sulphate. Once tubes had cooled, free ammonia was liberated by the addition of an excess of 40% NaOH and steam distilled into 30 ml of 4% boric acid indicator solution. Ammonia was then determined by titration against volumetric grade 0.1 M HCl using a Brand 25 ml digital burette. Nitrogen and protein content of the samples were calculated as follows:

$$\% \text{ nitrogen} = [(\text{sample titration} - \text{blank titration}) \times 0.1401]/\text{sample weight (g)}$$

$$\% \text{ protein} = \% \text{ nitrogen} \times 6.25$$

**Determination of carcass ash content**

Total ash in the freeze-dried samples was estimated by determining the non-combustible ash remaining after firing' samples at high temperatures using a Carbolite OAF 11/1 muffle furnace

Squat form silica crucibles were prefired (600˚C) for at least 2 h to stabilise weights. Crucibles were allowed to cool (<300˚C) and transferred to silica gel drying cabinets. Crucibles were then weighed, approximately 1 g of sample added and the crucible reweighed. A number of empty crucibles were also included. Crucibles were fired at 600˚C for 4 h to convert samples to ash and then allowed to cool (<300˚C) prior to transfer to silica gel drying cabinets. Final weights were determined when crucibles had fully cooled.

Percentage ash was determined by expressing the residual sample weight after firing as a percentage of the original sample weight. The average variation in blank crucible weight was -0.02 ± 0.04 mg (mean ± SEM, n=14) and individual weights varied by 0.5 mg or less.

**Plasma analyses**

Terminal blood samples via cardiac puncture were taken from the animals immediately after death into Sarstedt 4.5 ml lithium-heparin tubes (Cat No. 32.331). Tubes were then centrifuged at 1,500 g for 5 minutes at 4˚C and the plasma removed and immediately frozen on dry ice (as 3 aliquots per sample in sealed Eppendorf tubes). Plasma samples were stored at -75˚C until required for analysis. All determinations were performed on plasma samples that

had been thawed only once.

### Leptin assay

Plasma leptin concentration was determined using Assay Designs rat leptin enzyme immunometric assay kits (Cat No. 900-015) according to the manufacturer's regular protocol. Unknown samples were diluted 100 times using leptin assay buffer prior to assay in duplicate. Optical density at 450 nm was determined using a Molecular Devices VERSAmax tunable microplate reader and readings transferred to GraphPad Prism. A second order polynomial curve was fitted to the calibration data and unknown values extrapolated from this curve fit.

### NEFA assay

Non-esterified (or free) fatty acids (NEFA) were determined by modification of a commercial clinical analyser assay kit (Wako NEFA C ACS-ACOD method) to a 96-well format. The supplied oleic acid standard (1 mM) was diluted in deionised water to create a series of standard concentrations from 50 μM to 1000 μM. Plasma samples were assayed after a 2 times dilution in deionised water. Standards and diluted plasma samples were pipetted into the plates (Alpha Laboratories, flat bottomed FX9200) as 10 μl additions in duplicate. Assay plates and reagents A and B were preheated at 37˚C and the reaction started by adding 75 μl of reagent A followed by incubation at 37˚C. After exactly 10 minutes, 150 μl of reagent B was added and the plate incubated for a further 10 minutes after which optical density at 540 nm was determined using a Molecular Devices VERSAmax tunable microplate reader. Optical density readings were then transferred to GraphPad Prism and values for plasma samples extrapolated from a linear regression fit to the standard curve. The assay was linear up to the highest standard employed (1000 μM).

### Glycerol and triacylglycerol assay

Plasma triacylglycerol was determined by modification of a discrete assay kit (Sigma Triglyceride determination kit, Cat No. TR0100) to a 96-well format. The kit actually determines liberated glycerol following enzymic hydrolysis of triacylglycerol to free fatty acids and glycerol (1 mole glycerol per mole of triacylglycerol). For this reason, correction for the glycerol concentration originally present in the plasma is required. Plasma samples were, therefore, assayed in the absence (free plasma glycerol) or presence (total triacylglycerol) of lipoprotein lipase. Subtraction of free glycerol from total triacylglycerol concentration then derives the true triacylglycerol concentration.

A 2.5 mg/ml triolein equivalent glycerol standard was obtained from Sigma. The supplied Sigma mM conversion factor for this batch was 1.13 (ie 2.83 mM glycerol). A 10 mg/ml glycerol stock solution was prepared in saline and then diluted in saline to create a series of standard concentrations from 0.218 to 4.34 mM. Standards and plasma samples (undiluted) were pipetted into the 96-well plates as 10 μl additions in duplicate. Assay plates and reagents were preheated at 37˚C and the reaction started by adding 200 μl of either the glycerol or total triacylglycerol reagent (reconstituted as per manufacturer's protocol) followed by incubation at 37˚C for 5 minutes after which optical density at 540 nm was determined using a Molecular Devices VERSAmax tunable microplate reader. Optical density readings were then transferred to GraphPad Prism and unknown values extrapolated from a linear regression fit to the standard curve. The assay was linear up to the highest glycerol standard employed (4.34 mM). Subtraction of the glycerol concentration in the plasma samples from the total triacylglycerol concentration derived the true triacylglycerol concentration.

### Insulin assay

Plasma insulin concentration was determined using Mercodia ultrasensitive rat insulin ELISA kits according to the manufacturers 50 μl sample protocol. Unknown samples were diluted 20 times using the zero standard prior to assay in duplicate. Optical density at 450 nm was determined using a Molecular Devices VERSAmax tunable microplate reader and readings transferred to GraphPad Prism. A cubic spline curve was fitted to the calibration data and unknown values extrapolated from this curve fit.

### Results

<u>Chronic effects of COMPOUND B (10 and 30 mg/kg po) and COMPOUND A (10 and 30 mg/kg po) on the consumption of individual macronutrient sources</u>

In rats that had become obese after excessive consumption of a palatable "cafeteria" diet consisting of high-fat chow, ground dairy milk chocolate and ground roasted salted peanuts, repeated oral administration of COMPOUND B (10 and 30 mg/kg po) produced a moderate dose-related reduction in the consumption of these calorie-dense food

sources as reported in Figure 1. The reductions in food consumption COMPOUND B (30 mg/kg po only) were significant compared to the vehicle-treated control group on the first day of drug administration and daily food intake remained significantly lower than the control group on Days 2, 5, 6 and 7 and was non-significantly reduced until Day 14 (Figure 1). In comparison, COMPOUND B (10 mg/kg po) did not significantly alter food intake compared to the vehicle-treated control group on any day of drug treatment although daily food intakes were lower, than those of the control group on Days 1 to 3 (Figure 1).

In Figure 1) results are means (adjusted for differences between the food intakes of the different treatment groups at baseline (average of Days -6 to 0)) ± SEM (calculated from the residuals of the statistical model), n=9-10. Multiple comparisons against the vehicle-treated control group were by Dunnett's test. Significant differences from the control group are denoted by *p<0.05 (p levels have only been given at one level for clarity).

By comparison, COMPOUND A (10 and 30 mg/kg po) reduced food intake much more profoundly than COMPOUND B as shown in Figure 2. In comparison with the vehicle-treated control group the reduction of palatable food intake was significantly reduced by both the 10 and 30 mg/kg doses on the first day of drug administration and daily food intake remained significantly lower than the control group on Days 2 to 7 with the lower dose and on Days 2 to 10 and Day 15 for the higher dose (Figure 2).

In Figure 2) results are means (adjusted for differences between the food intakes of the different treatment groups at baseline (average of Days -6 to 0)) ± SEM (calculated from the residuals of the statistical model), n=9-10. Multiple comparisons against the vehicle-treated control group were by Dunnett's test. Significant differences from the control group are denoted by *p<0.05 (p levels have only been given at one level for clarity).

Chronic effects of COMPOUND A (10 and 30 mg/kg po) and COMPOUND B (10 and 30 mg/kg po) on the body weights of obese rats given access to highly Palatable, macronutrient food sources

The body weights of the vehicle-treated control group slowly increased throughout the study (Figures 3 and 4). Consistent with the moderate reductions of food intake described above, chronic oral administration of COMPOUND B (10 and 30 mg/kg po), evoked a small dose-related decrease in the body weights of these obese rats that were sustained throughout the duration of drug treatment (Figure 3). The body weight of animals given COMPOUND B (30 mg/kg po) did not reach the level of statistical significance compared to the control group until Day 6 and with a significant maximum decrease on Day 29 of 7.1% (Figure 3). The body weights of animals administered COMPOUND B (10 mg/kg po) tended to be lower than the vehicle-treated control group throughout the study (Figure 3), but these reductions did not reach statistical significance compared to the vehicle group with the exception of Day 3. At the end of the 28 day drug treatment period, the body weights of dietary-induced obese, female Wistar rats COMPOUND B (10 mg/kg po) were 4.0% lower than those of the vehicle-treated controls (Figure 3).

In Figure 3) results are means (adjusted for differences between the body weights of the different treatment groups at baseline (Day 1)) ± SEM (calculated from the residuals of the statistical model), n=9-10. Numbers represent % reduction compared to the control group on Day 29 (ie after 28 days of treatment). Multiple comparisons against the vehicle-treated control group were by Dunnett's test. Significant differences from the control group are denoted by *p<0.05 (p levels have only been given at one level for clarity).

Chronic administration of COMPOUND A (10 and 30 mg/kg po) produced much greater dose-related decreases in body weight than COMPOUND B (Figure 4). The body weights of animals given either dose of COMPOUND A were significantly lower than the control group on Day 2 (ie 24 h after the first dose of the drug) and on every other day of the drug treatment period (Figure 4). Thus, at the end of the 28 day drug treatment period, the body weights of dietary-induced obese, female Wistar rats given COMPOUND A (10 and 30 mg/kg po) were 12.0% and 19.0% lower, respectively, than those of the vehicle-treated controls (Figure 4).

In Figure 4) results are means (adjusted for differences between the body weights of the different treatment groups at baseline (Day 1)) ± SEM (calculated from the residuals of the statistical model), n=9-10. Numbers represent % reduction compared to the control group on Day 29 (ie after 28 days of treatment). Multiple comparisons against the vehicle-treated control group were by Dunnett's test. Significant differences from the control group are denoted by *p<0.05 (p levels have only been given at one level for clarity).

Chronic effects of COMPOUND A (10 and 30 mg/kg po) and COMPOUND B (10 and 30 mg/kg po) on the adiposity of obese rats given access to highly palatable, macronutrient food sources

The effects of chronic oral administration of COMPOUND B and COMPOUND A for 28 days on body composition are shown in Figures 5- 8 (fat, water, protein and ash content is expressed as g per rat).

Repeated administration with COMPOUND B (10 and 30 mg/kg po) evoked dose-related reductions in carcass fat, but this effect was only statistically significant at the higher dose (Figure 5). COMPOUND B (10 and 30 mg/kg po) produced a decreases in absolute body fat of 26.2 g and 11.1 g, respectively, compared to the vehicle-treated control

group (Figure 5).
In Figure 5 results are expressed as treatment group means (n=9-10 and adjusted for differences between the groups in body weight at baseline) and SEM (calculated from the residuals of the statistical model). Statistical comparisons were by ANCOVA (baseline body weight as covariate) followed by Dunnett's test. Significant differences are denoted by ***p<0.001, **p<0.01, *p<0.05.

By comparison, COMPOUND A (10 and 30 mg/kg po) produced much larger decreases in body fat of 42.8 g and 67.8 g, respectively, compared to the vehicle-treated control group (Figure 5)

COMPOUND B (10 and 30 mg/kg po) and COMPOUND A (10 and 30 mg/kg po) did not significantly alter total water (Figure 6), protein (Figure 7), or ash (Figure 8), content of the carcasses (expressed as weight per rat) with the exception of COMPOUND A (30 mg/kg po) which slightly, but significantly, decreased total water content.
In Figure 6) results are expressed as treatment group means (n=9-10 and adjusted for differences between the groups in body weight at baseline) and SEM (calculated from the residuals of the statistical model). Statistical comparisons were by ANCOVA (baseline body weight as covariate) followed by Dunnett's test. Significant differences are denoted by *p<0.05.
In Figures 7) and 8) results are expressed as treatment group means (n=9-10 and adjusted for differences between the groups in body weight at baseline) and SEM (calculated from the residuals of the statistical model). Statistical comparisons were by ANCOVA (baseline body weight as covariate) followed by Dunnett's test. No significant differences from control.

Consistent with the relative reductions in body fat shown in Figure 5, plasma leptin levels were decreased by 13.7% (non-significant) and 26.2% after chronic treatment with COMPOUND B (10 and 30 mg/kg po) and by 36.1%, 61.7% after COMPOUND A (10 and 30 mg/kg po) when compared to the vehicle-treated control group (Figure 9).
In Figure 9) results are expressed as treatment group means and SEM (all n=9-10). Analysis of covariance was used using baseline body weight and bleeding order (animal number) as covariate. The Rank transformation was used. Multiple comparisons against vehicle were by Dunnetts' test. Significant differences are denoted by *p.05 and ***p<0.001.

<u>Chronic effects of COMPOUND B (10 and 30 mg/kg po) and COMPOUND A (10 and 30 mg/kg po) on plasma NEFA concentrations in obese rats given access to highly palatable</u>, <u>macronutrient food sources</u>

The effects of repeated oral administration of COMPOUND B (10 and 30 mg/kg po) and COMPOUND A (10 and 30 mg/kg po) on plasma NEFA concentrations are shown in Figure 10.

COMPOUND B (30 mg/kg po) significantly decreased plasma NEFA levels by 24.4% compared to the vehicle-treated control group (Figure 10). The lower dose of this compound was, however, without effect on this lipid parameter. In contrast, despite the increased weight-loss and decreased adiposity resulting from repeated administration of COMPOUND A (10 and 30 mg/kg po), neither dose of this compound had any effect to reduce plasma NEFA levels (Figure 10).
In Figure 10) results are expressed as treatment group means and SEM (all n=9-10). Analysis of covariance was used using baseline body weight and bleeding order (animal number) as covariate. The Rank transformation was used. Multiple comparisons against vehicle were by Dunnetts' test. Significant differences are denoted by*p.05.

<u>Chronic effects of COMPOUND B (10 and 30 mg/kg po) and COMPOUND A (10 and 30 mg/kg po) on plasma triacylglycerol and glycerol concentrations in obese rats given access to highly palatable</u>, <u>macronutrient food sources</u>

The effects of repeated oral administration of COMPOUND B (10 and 30 mg/kg po) and COMPOUND A (10 and 30 mg/kg po) on plasma triacylglycerol and glycerol concentrations are shown in Figures 11 and 12.

COMPOUND B (30 mg/kg po) evoked highly significant reductions in the plasma concentrations of triacylglycerol of 52.1% ($p < 0.001$) and plasma glycerol levels of 38.9% ($p < 0.01$), although treatment with the lower dose of 10 mg/kg was without effect on these lipid parameters (Figures 11 and 12).

COMPOUND A (30 mg/kg po) also significantly reduced plasma triacylglycerol concentrations by 42% ($p < 0.05$) and plasma glycerol levels by 33.6% ($p < 0.05$) (Figures 11 and 12). In spite of the fact that COMPOUND A (10 mg/kg po) induced a greater weight-loss and reduction in adiposity than COMPOUND B (30 mg/kg po), it had no significant effect on plasma triacylglycerol or glycerol levels (Figures 11 and 12).
In Figures 11) and 12) results are expressed as treatment group means and SEM (all n=9-10). Analysis of covariance was used using baseline body weight and bleeding order (animal number) as covariate. The Rank transformation was used. Multiple comparisons against vehicle were by Dunnetts' test. Significant differences are denoted by *p.05 and ***p<0.001.

<u>Chronic effects of COMPOUND B (10 and 30 mg/kg po) and COMPOUND A (10 and 30 mg/kg po) on plasma insulin concentrations in obese rats given access to highly palatable, macronutrient food sources</u>

The effects of COMPOUND A (10 and 30 mg/kg po) and COMPOUND B (10 and 30 mg/kg po) on plasma

insulin levels are shown in Figure 13.

COMPOUND B (10 and 30 mg/kg po) reduced plasma insulin levels by 15% to 28% compared to the control group (Figure 13).

COMPOUND A (10 and 30 mg/kg po) significantly decreased plasma insulin levels by 38.9% and 41.8%, respectively compared to the vehicle-treated control group (Figure 13).

In Figure 13) results are expressed as treatment group means and SEM (all n=9-10). Analysis of covariance was used using baseline body weight and bleeding order (animal number) as covariate. The Rank transformation was used. Multiple comparisons against vehicle were by Dunnetts' test. Significant differences are denoted by **p<0.01, ***p<0.001.

## DISCUSSION

In the present invention, it has surprisingly been demonstrated that (S)-N-piperidinyl-5-(4-chlorophenyl)-1-(2,4-dichlorophenyl)-4,5-dihydro-1H-pyrazole-3-carboxamide (Compound B) is unexpectedly beneficial as a medicament for the prophylaxis and/or treatment of dyslipidaemia and related metabolic disorders including obesity, central adiposity, insulin resistance/impaired glucose tolerance (pre-diabetes), Type 2 diabetes and Metabolic Syndrome that result from the excessive consumption or binge eating of highly palatable food sources.

This invention is surprising as demonstrated by 4 findings:

1. Although its enantiomer COMPOUND A has an $IC_{50}$ value of 22 nM, COMPOUND B lacks a pharmacologically relevant affinity for the rat CB1 receptor, ie a $IC_{50}$ value of 813 nM.

2. COMPOUND B evokes decreases the excessive consumption of high fat foods (exemplified by high-fat chow), high carbohydrate foods and sweets (exemplified by ground dairy milk chocolate) and savoury high protein foods (exemplified by ground roasted salted peanuts) when they were freely available to the animals as individual "cafeteria" macronutrient sources. The reduction in palatable food sources results in reductions of various plasma lipids, ie NEFAs, triacylglycerol and glycerol, weight-loss mediated by fat-loss and modest reductions in plasma insulin. Since COMPOUND B lacks affinity for the rat CB1 receptor, these benefits cannot be mediated through an inhibition of CB1 receptor function, and are, therefore, mediated by a novel and as yet unknown mechanism.

3. In a comparison with the CB1 receptor antagonist, COMPOUND A, COMPOUND B was the only compound that decreased the plasma concentrations of NEFAs.

4. COMPOUND B (30 mg/kg) evoked greater reductions in plasma triacylglycerol and glycerol than COMPOUND A (10 mg/kg) even though the former produced less weight-loss than the latter.

The implication from points 3 and 4 is COMPOUND B has a beneficial effect on plasma lipids which is greater than that accounted for by weight-loss alone.

In this invention, the use of COMPOUND B, in the prophylaxis and treatment of dyslipidaemia resulting from the excessive consumption or binge eating of highly palatable food sources has been claimed.

Dyslipidaemia exists as a disease entity in its own right as well as being a core component of the various definitions of the Metabolic Syndrome. Thus, dyslipidaemia comprises heterozygous familial hypercholesterolaemia (FH), homozygous familial hypercholesterolaemia (FH), familial defective apolipoprotein B-100 (FDB), polygenic hypercholesterolaemia hypertriglyceridaemia including familial combined hyperlipidaemia, familial hypertriglyceridaemia and familial dysbetalipoproteinaemia, low HDL-cholesterol (with or without hypertriglyceridaemia), diabetic dyslipidaemia (ie atherogenic dyslipidaemia in persons with Type 2 diabetes), elevated LDL-cholesterol and atherogenic dyslipidaemia. Other secondary dyslipidaemias include, but are not limited to, those evoked by hypothyroidism, nephrotic syndrome and other renal disorders, obstructive liver disease and protease-inhibitor induced dyslipidaemia.

Within the various definitions of the Metabolic Syndrome that are given below lipid abnormalities are specifically defined as follows.

<u>World Health Organisation (WHO) (1999)</u>

- Raised plasma triglycerides (≥ 1.7 mmol/L; 150 mg/dL).
  Low plasma HDL-cholesterol (< 0.9 mmol/L, 35 m/dL men; < 1.0 mmol/L, 39 mg/dL women).

National Cholesterol Education Programme (NCEP) Adult Treatment Panel (ATP) III (2002)

- Raised plasma triglycerides (≥ 1.7 mmol/L; 150 mg/dL).
Low plasma HDL-cholesterol (< 1.03 mmol/L, 40 mg/dL men; < 1.29 mmol/L, 50 mg/dL women).

International Diabetes Federation (IDF) (2005)

Raised serum triglycerides (≥ 1.7 mmol/L; 150 mg/dL) or specific treatment for this lipid abnormality.
Reduced plasma HDL-cholesterol (< 1.03 mmol/L, 40 mg/dL men; 1.29 mmol/L, 50 mg/dL women) or specific treatment for this abnormality.
As also stated in the NCEP ATP III Guidelines (2002), the necessity to treat dyslipidaemia in patients is defined not only by the serum or plasma concentrations of individual lipids, but also by the coexistence of lipid abnormalities with other cardiovascular risk factors and/or related disorders, eg hypertension, cardiovascular disease and Type 2 diabetes, the patient's medical history, also whether or not the patient has a history of cerebrovascular or cardiovascular adverse events, eg myocardial infarction, congestive heart failure, angina and/or haemorrhagic or thromboembolic stroke. Such clinical factors are well known to those skilled in the art and are taken into account in the decision whether or not to prescribe medications to treat dyslipidaemia.
All of these indications are claimed in this application.
It is also well known that obesity and visceral adiposity are important metabolic consequences of the excessive consumption of highly palatable calorie-dense foods (Zephier et al, 1997; International Diabetes Federation, 2003; Grundy, 2004; Deedwania, 2004), and in some instances, from cravings for and binge eating of specific palatable food sources (White et al, 2002; Phelan & Whadden, 2002; Yanovski, 2003; White & Grilo, 2005). The data presented show that COMPOUND B is unexpectedly efficacious in reducing obesity and adiposity resulting from the consumption of these highly palatable food sources and the use of COMPOUND B as a medicament to treat or prevent obesity and visceral adiposity resulting from the excessive consumption or binge eating of highly palatable food sources is also claimed.
Dyslipaemia, with or without obesity, resulting from the excessive consumption of highly palatable calorie-dense foods (Zephier et al, 1997; International Diabetes Federation, 2003; Grundy, 2004; Deedwania, 2004), and in some instances, from cravings for and binge eating of specific palatable food sources (White et al, 2002; Phelan & Whadden, 2002; Yanovski, 2003; White & Grilo, 2005) is major cause of insulin resistance and a key driver of the progression of pre-diabetes (insulin resistance and/or impaired glucose tolerance) to Type 2 diabetes (Boden & Laakso, 2004; Bays et al, 2004; IDF, 2005). Furthermore, there is also a high degree of association between Type 2 diabetes and dyslipidaemia whereby the latter is characterised by raised plasma levels of small, dense LDL-cholesterol particles, elevated triglycerides and low concentrations of HDL-cholesterol particles (Boden & Laakso, 2004). Since COMPOUND B has been unexpectedly shown to improve dyslipidaemia resulting from the over-consumption of highly palatable food sources, and in addition, to reduce plasma insulin levels, the use of COMPOUND B as a medicament to treat or prevent insulin resistance/impaired glucose tolerance or Type 2 diabetes resulting from the excessive consumption or binge eating of highly palatable food sources is also claimed.
The excessive consumption of highly palatable calorie-dense foods (Zephier et al, 1997; International Diabetes Federation, 2003; Grundy, 2004; Deedwania, 2004), and in some instances, from cravings for and binge eating of specific palatable food sources (White et al, 2002; Phelan & Whadden, 2002; Yanovski, 2003; White & Grilo, 2005) is a major cause of the increased prevalence of the Metabolic Syndrome. In this cluster of cardio-metabolic risk factors, obesity or central adiposity, lipid abnormalities, (raised serum triglycerides, low serum HDL-cholesterol) and impaired glucose tolerance or Type 2 diabetes are core symptoms of the Metabolic Syndrome, irrespective of whether a diagnosis of is made according to the criteria defined by the World Health Organisation (WHO, 1999), the National Cholesterol Education Programme - Third Adult Treatment Panel (NCEP ATP III, 2001) or the International Diabetes Federation (IDF, 2005). The cafeteria fed obese rats employed in these experiments are obese, dyslipidaemic and insulin resistant making them an excellent surrogate model for the human Metabolic Syndrome resulting from the over-consumption of highly palatable food sources. Since COMPOUND B has been unexpectedly shown to reduce obesity and adiposity, to decrease plasma NEFAs, triacylglycerol, glycerol and insulin levels in this animal model, the use of COMPOUND B as a medicament to treat or prevent the Metabolic Syndrome resulting from the excessive consumption or binge eating of highly palatable food sources is also claimed.
The beneficial effect of COMPOUND B to decrease plasma NEFAs, tracylglycerol, glycerol and insulin levels is greater than that which can be accounted for by weight-loss alone. Hence, the results also demonstrate that COMPOUND B will be useful as a medicament to treat or prevent dyslipidaemia and/or insulin resistance/impaired glucose tolerance or Type 2 diabetes or Metabolic Syndrome resulting from the excessive consumption of highly palatable calorie-dense foods or from cravings/binge eating of specific palatable food sources in subjects in whom these metabolic disturbances are not associated with being obese or overweight.

Together, COMPOUND A and COMPOUND B are the enantiomers which comprise the racemic compound, (rac)-N-piperidiny)-5-(4-chlorophenyl)-1-(2,4-dichlorophenyl)-4,5-dihydro-1H-pyrazole-3-carboxamide which itself is a pyrazoline that has been claimed previously as medicaments for the treatment and/or prophylaxis of obesity. COMPOUND A is a CB1 antagonist with demonstrated benefits on obesity and/or central adiposity and/or dyslipidaemia and/or insulin resistance/impaired glucose tolerance or Type 2 diabetes and/or Metabolic Syndrome resulting from the excessive consumption of highly palatable calorie-dense foods or from cravings/binge eating of specific palatable food sources, whilst COMPOUND B provides additional weight-loss dependent and weight-loss-independent beneficial effects on these cardio-metabolic risk factors through a novel, as and yet unidentified, pharmacological mechanism. On this basis, (rac)-N-piperidinyl-5-(4-chlorophenyl)-1-(2,4-dichlorophenyl)-4,5-dihydro-1H-pyrazole-3-carboxamide will be unexpectedly advantaged as a medicament in non-obese, overweight or obese subjects, who are suffering from obesity and/or central adiposity and/or dyslipidaemia and/or insulin resistance/impaired glucose tolerance or Type 2 diabetes and/or Metabolic Syndrome, when it is a consequence of the excessive consumption of highly palatable calorie-dense foods or from cravings for and binge eating of specific palatable food sources.

## REFERENCES

American Heart Association. Third Report of the National Cholesterol Education Program (NCEP) Expert Panel on Detection Evaluation, and Treatment of High Blood Cholesterol in Adults (Adult Treatment Panel III) Final Report. Circulation 2002;106:3143-3420.

Bays H, Mandarino L, DeFronzo RA. Mechanisms of endocrine disease. Role of the adipocyte, free fatty acids, and ectopic fat in pathogenesis of Type 2 diabetes mellitus: peroxisomal proliferator-activated receptor agonists provide a rational therapeutic approach. J Clin Endocrine Metab 2004;89:463-478.

Boden G, Laakso M. Lipids and glucose in Type 2 diabetes. What is the cause and effect? Diabetes Care 2004;27:2253-2259.

Deedwania PC. Metabolic Syndrome and vascular disease: is nature or nurture leading the new epidemic of cardiovascular disease? Am Heart Ass Circulation 2004;109:2-4.

Govaerts SJ, Hermans E, Lambert DM. Comparison of cannabinoid ligand affinities and efficacies in murine tissues and in transfected cells expressing human recombinant cannabinoid receptors. Eur J Pharm Sci. 2004; 23: 233 - 43.

Grundy SM. Obesity, Metabolic Syndrome, and cardiovascular disease. J Clin Endocrinol Metab. 2004;89: 2595-2600.

IDF consensus worldwide definition of the Metabolic Syndrome. IDF 2005. www.idf.org.

International Diabetes Federation (IDF). Diabetes Atlas 2003,

Phelan S, Wadden TA. Combining behavioural and pharmacological treatments for obesity. Obes Res 2002; 10:560-574.

White MA, Grilo CM. Psychometric properties of the Food Craving Inventory among obese patients with binge eating disorder. Eat Behav 2005;6:239-245.

White MA, Whisenhunt BL, Williamson DA, Greenway FL, Netemeyer RG. Development and validation of the Food-Craving Inventory. Obes Res 2002;10:107-114.

WHO: World Health Organization. Definition, diagnosis and classification of diabetes mellitus and its complications. Report of a WHO consultation 1999.

Yanovski S. Sugar and fat: cravings and aversions. J Nutr 2003;133:835S-837S.

Zephier EM, Ballew C, Mokdad A, Mendlein J, Smith C, Yeh JL, Lee E, Welty TK, Howard B. Intake of nutrients related to cardiovascular disease risk among three groups of American Indians: the Strong Heart Dietary Study. Prev Med 1997;26:508-515.

## Claims

1. Nonracemic mixture of (S)-N-piperidinyl-5-(4-chlorophenyl)-1-(2,4-dichlorophenyl)-4,5-dihydro-1H-pyrazole-3-carboxamide and (R)-N-piperidinyl-5-(4-chlorophenyl)-1-(2,4-dichlorophenyl)-4,5-dihydro-1 H-pyrazole-3-carboxamide, their respective salts, polymorphs or solvates, especially hydrates.

2. Nonracemic mixture according to claim 1, **characterized in that** (S)-N-piperidinyl-5-(4-chlorophenyl)-1-(2,4-dichlorophenyl)-4,5-dihydro-1H-pyrazole-3-carboxamide is present in the mixture in an amount X of between 99 to 51 percent by weight, preferably 95 to 55 percent by weight, more

preferably 90 to 60 percent by weight, most preferably 80 to 60 percent by weight and (R)-N-piperidinyl-5-(4-chlorophenyl)-1-(2,4-dichlorophenyl)-4,5-dihydro-1H-pyrazole-3-carboxamide is present in the mixture in an amount Y of between 01 to 49 percent by weight, preferably 05 to 45 percent by weight, more preferably 10 to 40 percent by weight, most preferably 20 to 40 percent by weight, with **X+Y** being 100.

**3.** Nonracemic mixture according to claim 1, **characterized in that** (R)-N-piperidinyl-5-(4-chlorophenyl)-1-(2,4-dichlorophenyl)-4,5-dihydro-1H-pyrazole-3-carboxamide is present in the mixture in an amount X of between 99 to 51 percent by weight, preferably 95 to 55 percent by weight, more preferably 90 to 60 percent by weight, most preferably 80 to 60 percent by weight and (S)-N-piperidinyl-5-(4-chlorophenyl)-1-(2,4-dichlorophenyl)-4,5-dihydro-1H-pyrazole-3-carboxamide is present in the mixture in an amount Y of between 01 to 49 percent by weight, preferably 05 to 45 percent by weight, more preferably 10 to 40 percent by weight, most preferably 20 to 40 percent by weight, with X+Y being 100.

**4.** Nonracemic mixture according to any one of claim 1, 2 or 3 **characterized in that** one of (S)-N-piperidinyl-5-(4-chlorophenyl)-1-(2,4-dichlorophenyl)-4,5-dihydro-1H-pyrazole-3-carboxamide or (R)-N-piperidinyl-5-(4-chlorophenyl)-1-(2,4-dichlorophenyl)-4,5-dihydro-1H-pyrazole-3-carboxamide is present in in form of its base or hydrochloride, preferably both are in form of their respective bases or hydrochlorides, most preferably both are in form of their respective bases.

**5.** Medicament comprising at least the nonracemic mixture according to any of claims 1 to 4 and optionally one or more pharmaceutically acceptable excipients.

**6.** Use of the non-racemic mixture according to any of claim 1 to 4 for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**7.** Use of the non-racemic mixture according to any of claim 1 to 4 for the preparation of a medicament for the prophylaxis and/or treatment of food intake disorders, preferably bulimia, anorexia, cachexia, obesity, type II diabetes mellitus (non-insuline dependent diabetes mellitus), more preferably obesity, type II diabetes, metabolic syndrome and/or dyslipidaemia.

**8.** Pharmaceutical formulation comprising

- the mixture according to any one of claims 1 to 4 and (S)-N-piperidinyl-5-(4-chlorophenyl)-1-(2,4-dichlorophenyl)-4,5-dihydro-1H-pyrazole-3-carboxamide; or
- the mixture according to any one of claims 1 to 4 and (R)-N-piperidinyl-5-(4-chlorophenyl)-1-(2,4-dichlorophenyl)-4,5-dihydro-1H-pyrazole-3-carboxamide; or
- the mixture according to any one of claims 1 to 4 and (rac) -N-piperidinyl-5-(4-chlorophenyl)-1-(2,4-dichlorophenyl)-4,5-dihydro-1H-pyrazole-3-carboxamide; or
- (S)-N-piperidinyl-5-(4-chlorophenyl)-1-(2,4-dichlorophenyl)-4,5-dihydro-1H-pyrazole-3-carboxamide and (R)-N-piperidinyl-5-(4-chlorophenyl)-1-(2,4-dichlorophenyl)-4,5-dihydro-1H-pyrazole-3-carboxamide; in separated compartments of the pharmaceutical formulation.

**9.** Pharmaceutical formulation according to claim 8, charactzerized in that the separated compartments are layers or pellets.

**10.** Use of (S)-N-piperidinyi-5-(4-ch)orophenyl)-1-(2,4-dich)orophenyl)-4,5-dihydro-1H-pyrazole-3-carboxamide its respective salts, polymorphs or solvates, especially hydrates for the preparation of a medicament for the prophylaxis and/or treatment of dyslipidaemia and other metabolic disturbances that result from the ecessive consumption of highly palatable macronutrient food sources.

**11.** Use according to claim 10, wherein the other metabolic disturbances that result from the ecessive consumption of highly palatable macronutrient food sources are selected from obesity, central adiposity, insulin resistance/impaired glucose tolerance (pre-diabetes), Type 2 diabetes and Metabolic Syndrome; especially metabolic syndrome or diabetes type II.

Figure 1

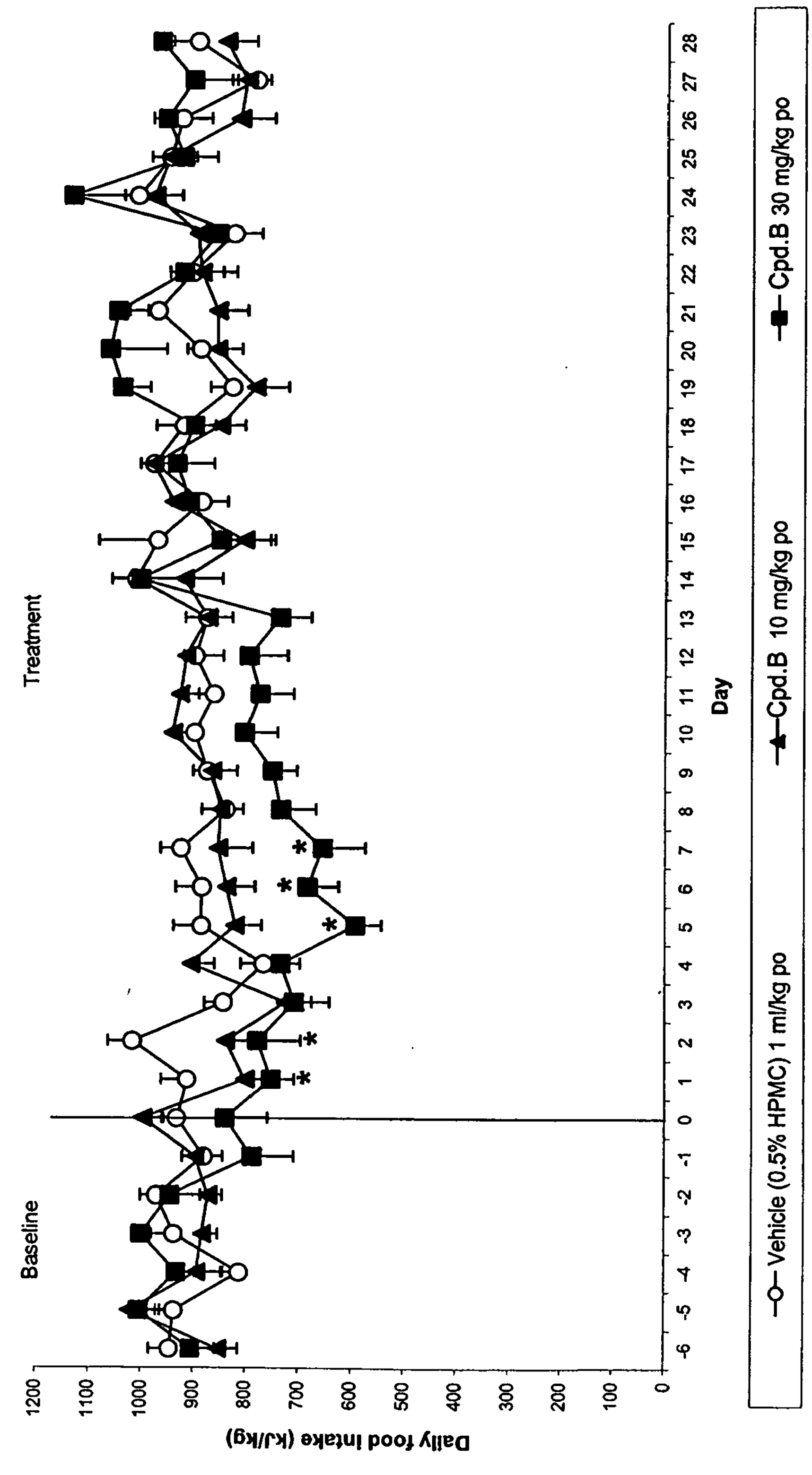

Baseline
Treatment
Daily food intake (kJ/kg)
Day
Vehicle (0.5% HPMC) 1 ml/kg po
Cpd.B 10 mg/kg po
Cpd.B 30 mg/kg po

Figure 2

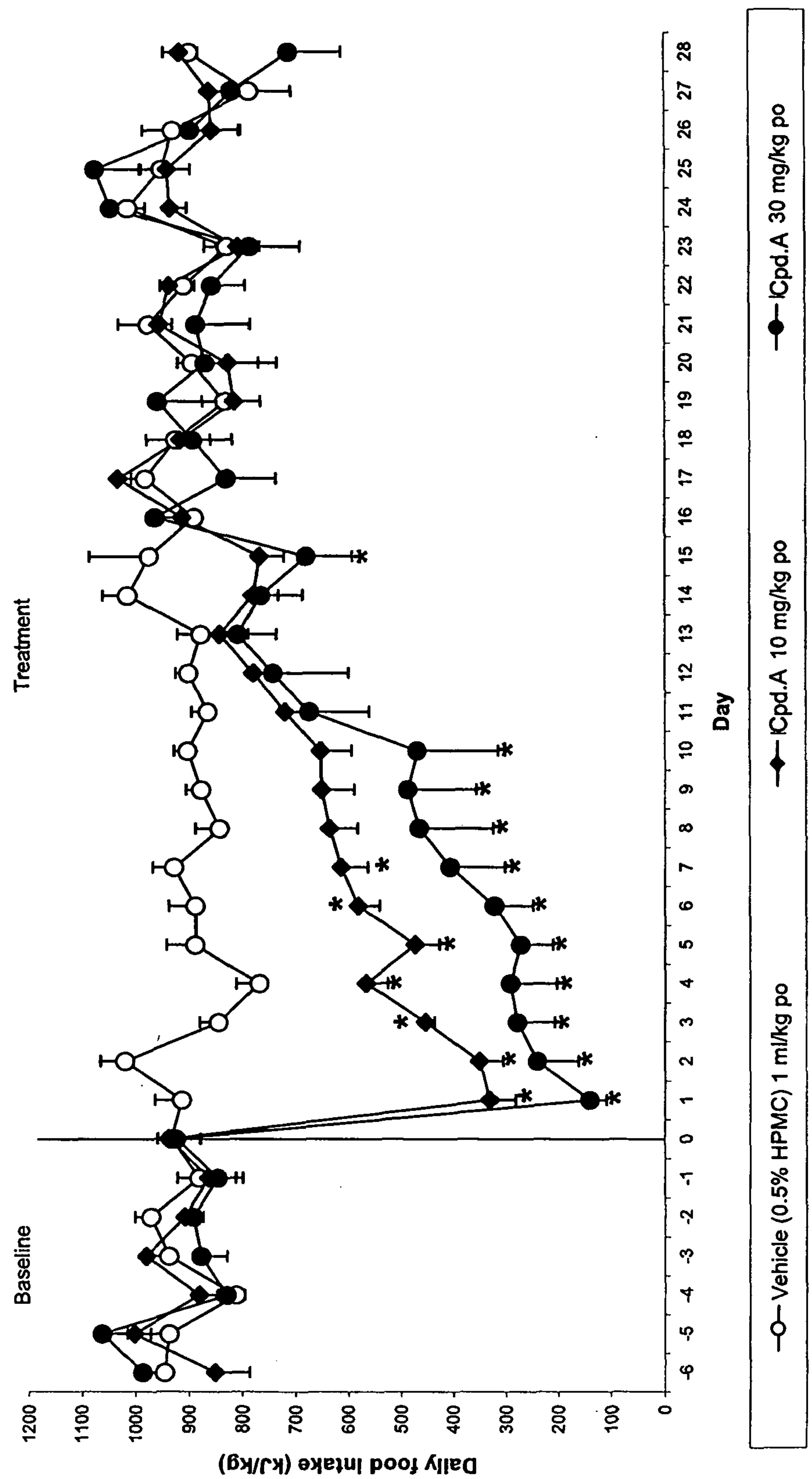
Baseline
Treatment
Daily food intake (kJ/kg)
0
100
200
300
400
500
600
700
800
900
1000
1100
1200
-6 -5 -4 -3 -2 -1 0 1 2 3 4 5 6 7 8 9 10 11 12 13 14 15 16 17 18 19 20 21 22 23 24 25 26 27 28
Day
Vehicle (0.5% HPMC) 1 ml/kg po
Cpd.A 10 mg/kg po
Cpd.A 30 mg/kg po

Figure 3

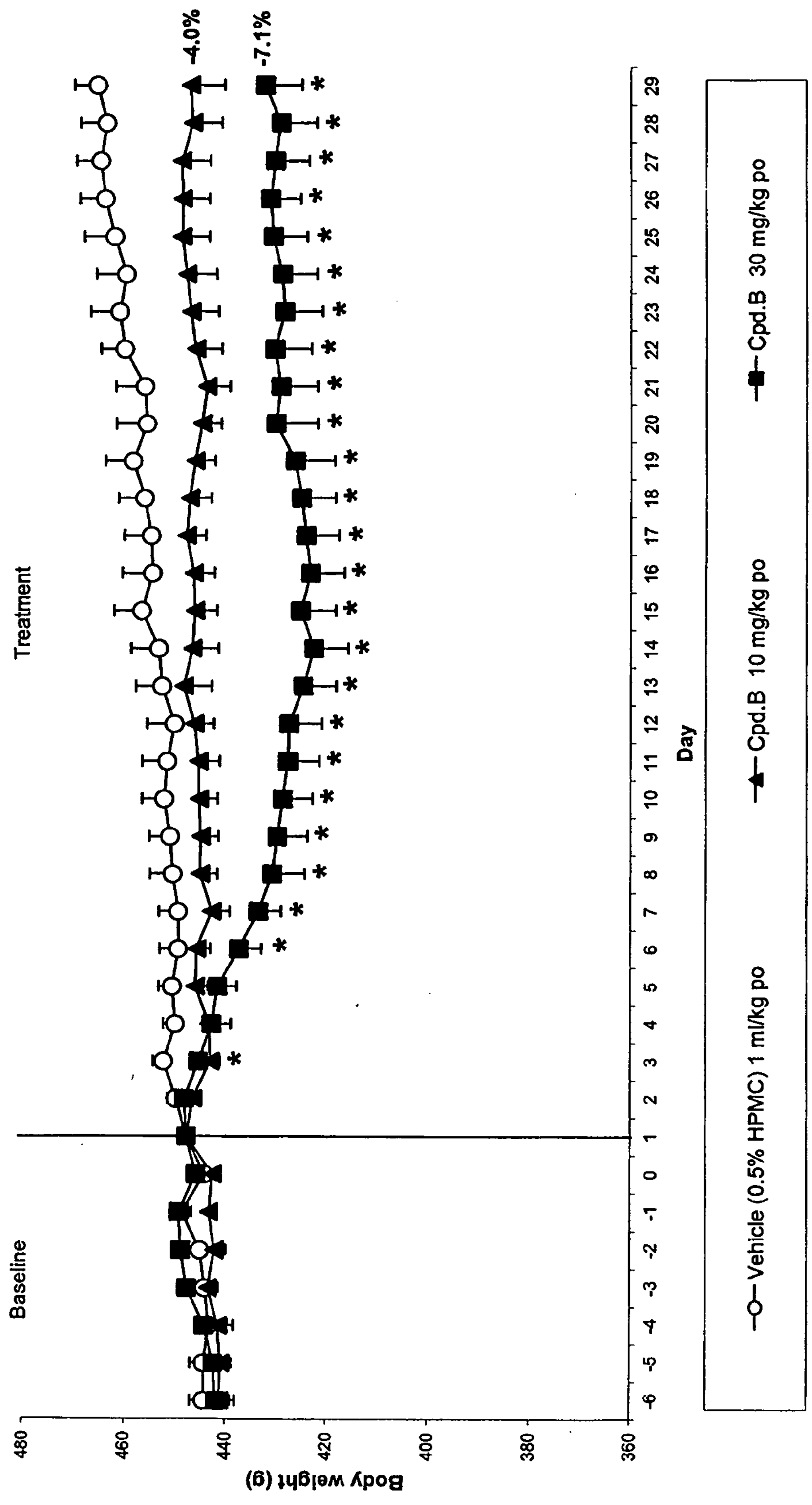
Baseline
Treatment
Body weight (g)
Day
-4.0%
-7.1%
Vehicle (0.5% HPMC) 1 ml/kg po
Cpd.B 10 mg/kg po
Cpd.B 30 mg/kg po

Figure 4

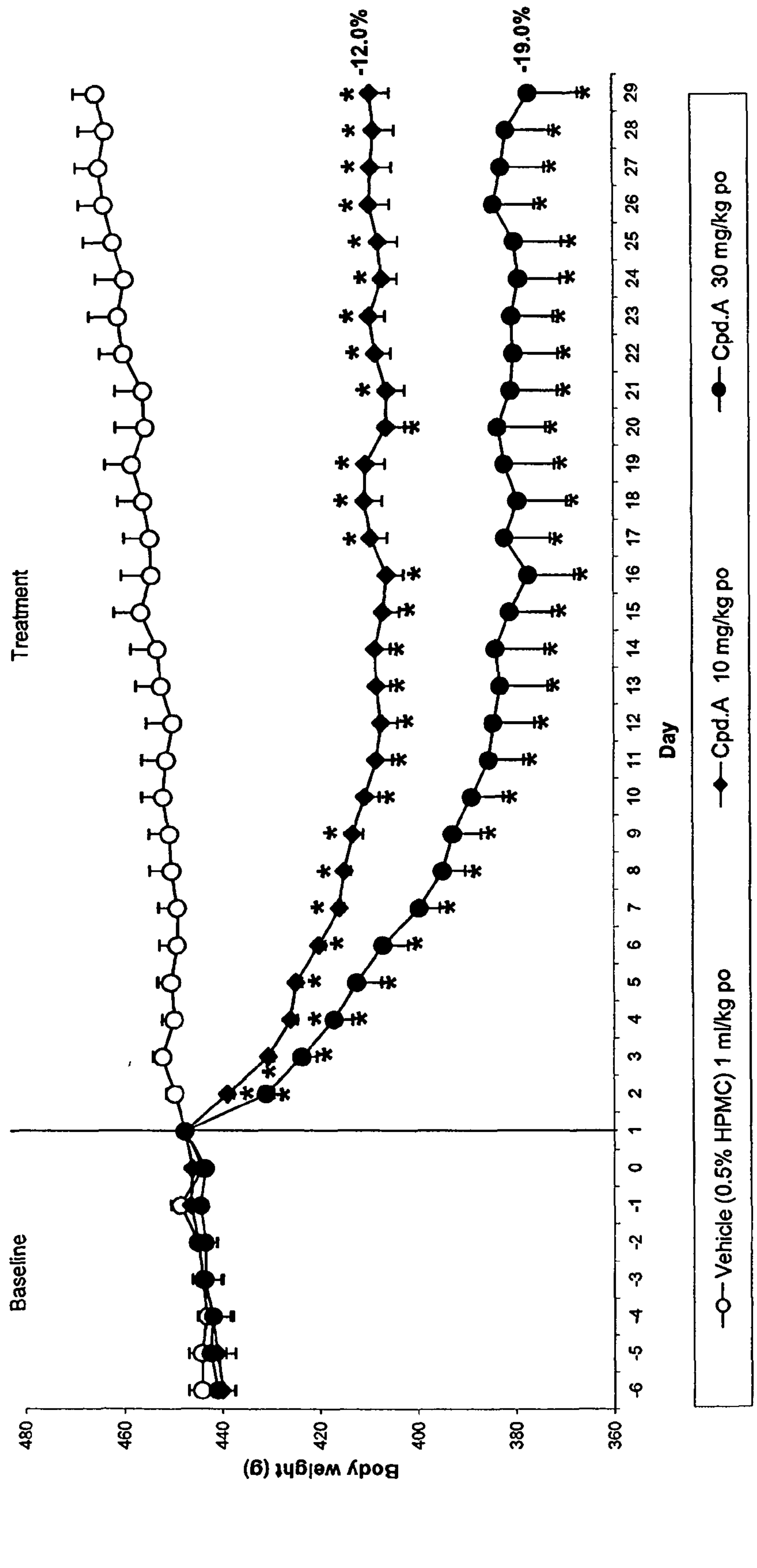
Baseline
Treatment
Body weight (g)
Day
-12.0%
-19.0%
Vehicle (0.5% HPMC) 1 ml/kg po
Cpd.A 10 mg/kg po
Cpd.A 30 mg/kg po

Figure 5:

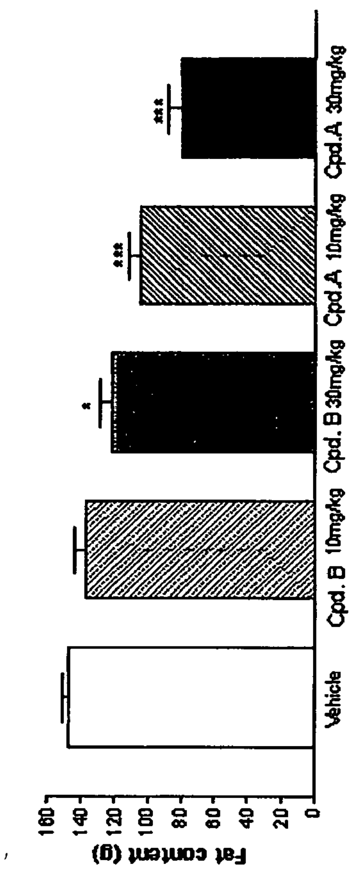
Fat content (g)
160
140
120
100
80
60
40
20
0
Vehicle
Cpd. B 10mg/kg
Cpd. B 30mg/kg
Cpd.A 10mg/kg
Cpd.A 30mg/kg
*
***
***

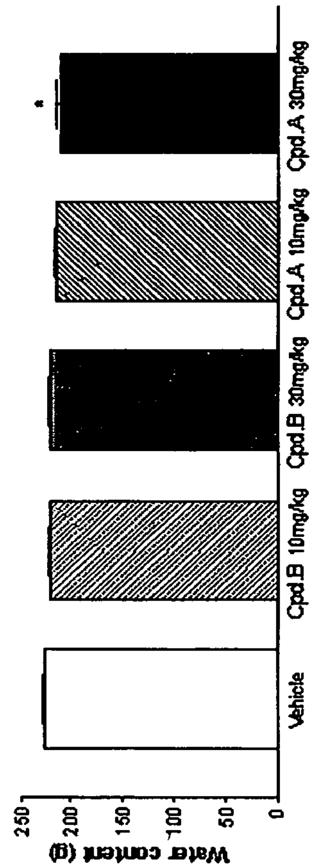
Water content (g)
250
200
150
100
50
0
Vehicle
Cpd.B 10mg/kg
Cpd.B 30mg/kg
Cpd.A 10mg/kg
Cpd.A 30mg/kg
*

Figure 6:

Figure 7:

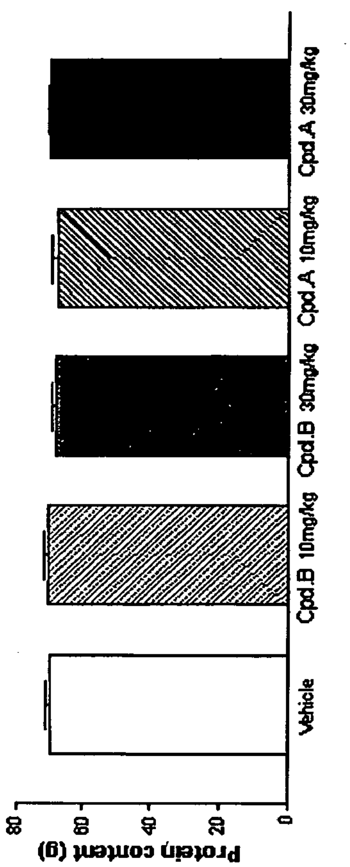
Protein content (g)
80
60
40
20
0
Vehicle
Cpd.B 10mg/kg
Cpd.B 30mg/kg
Cpd.A 10mg/kg
Cpd.A 30mg/kg

Figure 8:

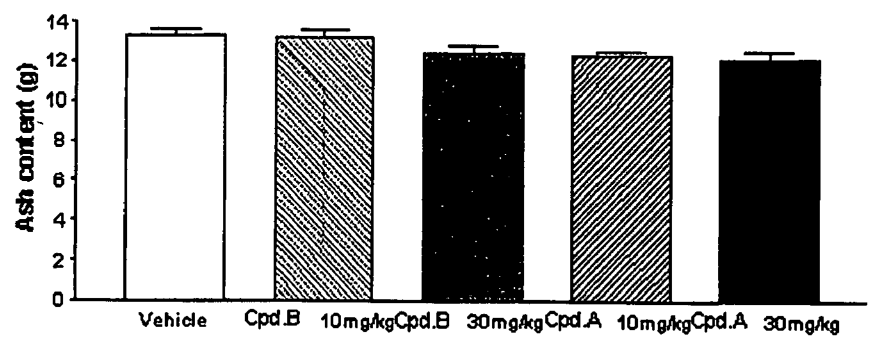
Ash content (g)
14
12
10
8
6
4
2
0
Vehicle
Cpd.B 10mg/kg
Cpd.B 30mg/kg
Cpd.A 10mg/kg
Cpd.A 30mg/kg

Figure 9:

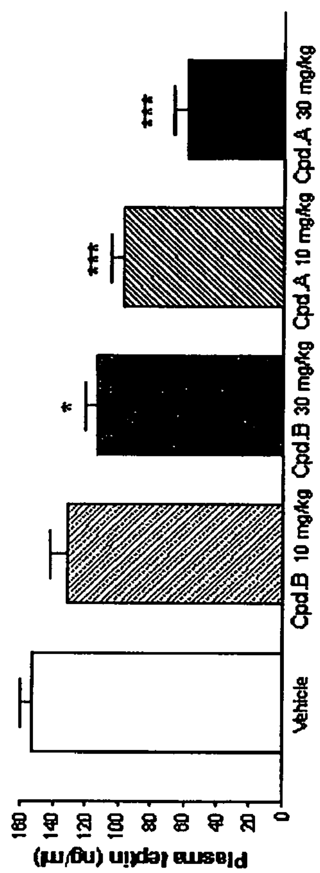
Plasma leptin (ng/ml)
180
140
120
100
80
60
40
20
0
Vehicle
Cpd.B 10 mg/kg
Cpd.B 30 mg/kg
Cpd.A 10 mg/kg
Cpd.A 30 mg/kg
*
***
***

Figure 10:

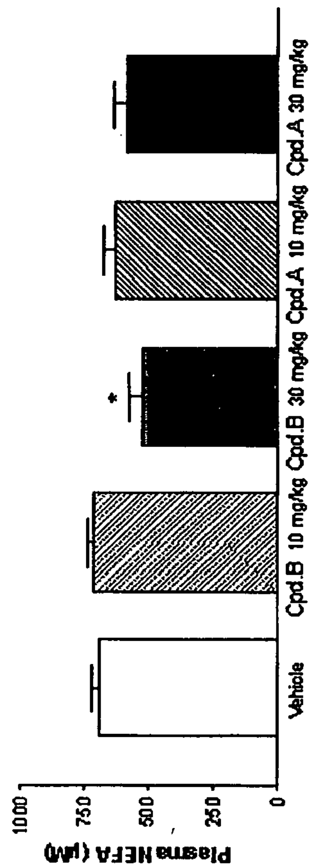
Plasma NEFA (µM)
1000
750
500
250
0
Vehicle
Cpd.B 10 mg/kg
Cpd.B 30 mg/kg
Cpd.A 10 mg/kg
Cpd.A 30 mg/kg
*

Figure 11:

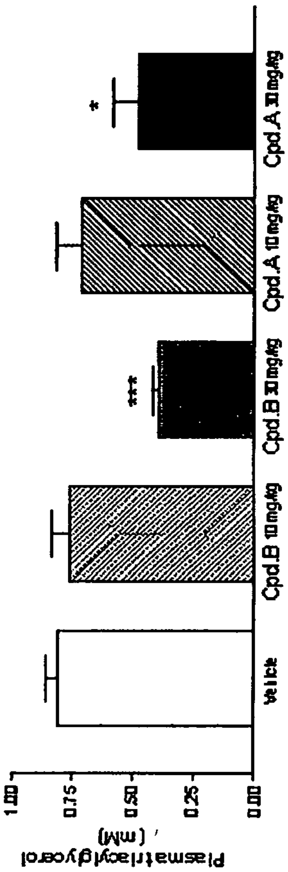
Plasma triacylglycerol (mM)
1.00
0.75
0.50
0.25
0.00
Vehicle
Cpd.B 10 mg/kg
Cpd.B 30 mg/kg
Cpd.A 10 mg/kg
Cpd.A 30 mg/kg
***
*

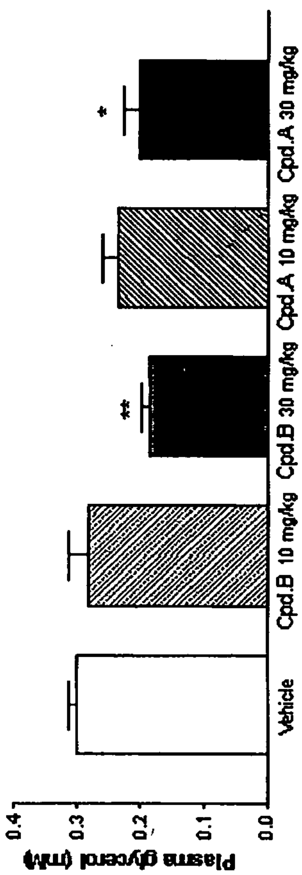
Plasma glycerol (mM)
0.4
0.3
0.2
0.1
0.0
Vehicle
Cpd.B 10 mg/kg
Cpd.B 30 mg/kg
Cpd.A 10 mg/kg
Cpd.A 30 mg/kg
**
*

Figure 12:

Figure 13:

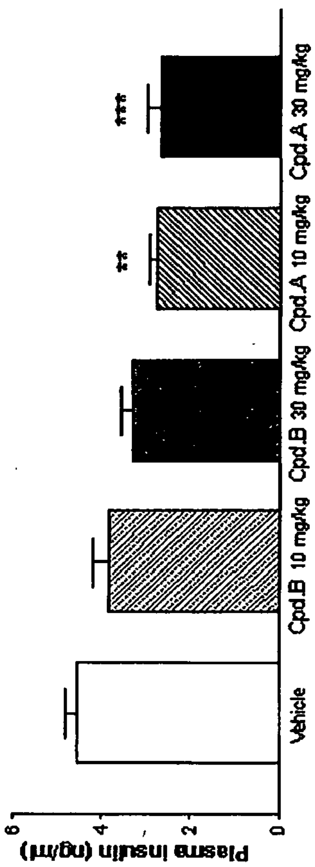
Plasma insulin (ng/ml)
0
2
4
6
Vehicle
Cpd.B 10 mg/kg
Cpd.B 30 mg/kg
Cpd.A 10 mg/kg
Cpd.A 30 mg/kg
**
***

European Patent Office

# EUROPEAN SEARCH REPORT

Application Number
EP 07 38 4005

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/077909 A (ESTEVE LABOR DR [ES]; CUBERES ALTISEN ROSA [ES]; GUITIERREZ SILVA BONI) 25 August 2005 (2005-08-25) * page 49 * ----- | 1-11 | INV. C07D231/06 A61K31/415 A61P3/06 |
| Y | WO 2005/074920 A (SOLVAY PHARM BV [NL]; LANGE JOSEPHUS H M [NL]; KRUSE CORNELIS G [NL];) 18 August 2005 (2005-08-18) * page 14 * ----- | 1-11 | |
| A | ADAMS J ET AL: "Recent advances in the cannabinoids" EXPERT OPINION ON THERAPEUTIC PATENTS, ASHLEY PUBLICATIONS, GB, vol. 12, no. 10, 2002, pages 1475-1489, XP002353808 ISSN: 1354-3776 * the whole document * ----- | 1-11 | |
| E | EP 1 743 892 A (ESTEVE LABOR DR [ES]) 17 January 2007 (2007-01-17) *page 11* *paragraph [0084]* ----- | 10,11 | TECHNICAL FIELDS SEARCHED (IPC): C07D |

The present search report has been drawn up for all claims

1

| Place of search | Date of completion of the search | Examiner |
|---|---|---|
| Munich | 18 May 2007 | Baston, Eckhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 38 4005

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2007

| Patent document cited in search report | Publication date | Patent family member(s) | Publication date |
|---|---|---|---|
| WO 2005077909 A | 25-08-2005 | AU 2005212817 A1 | 25-08-2005 |
| | | CA 2556568 A1 | 25-08-2005 |
| | | KR 20060135815 A | 29-12-2006 |
| WO 2005074920 A | 18-08-2005 | AU 2005210163 A1 | 18-08-2005 |
| | | CA 2552940 A1 | 18-08-2005 |
| | | KR 20060131861 A | 20-12-2006 |
| | | MX PA06008593 A | 28-08-2006 |
| EP 1743892 A | 17-01-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005077909 A
- WO 2005077911 A


### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992, 459
- *International Diabetes Federation,* 2003
- **ECKEL et al.** *The Lancet,* 2005, vol. 365, 1415-1428
- **ALBERTI et al.** *Diabet. Med.,* 1998, vol. 15, 539-53
- *JAMA,* 2001, vol. 285, 2486-97
- **PAGOTTO ; PASQUALI.** *The Lancet,* 2005, vol. 365, 1363, 1364
- *International Diabetes Federation,* 2005
- *Circulation,* 2002, vol. 106, 3143-3420
- **BAYS H ; MANDARINO L ; DEFRONZO RA.** Mechanisms of endocrine disease. Role of the adipocyte, free fatty acids, and ectopic fat in pathogenesis of Type 2 diabetes mellitus: peroxisomal proliferator-activated receptor agonists provide a rational therapeutic approach. *J Clin Endocrine Metab,* 2004, vol. 89, 463-478
- **BODEN G ; LAAKSO M.** Lipids and glucose in Type 2 diabetes. What is the cause and effect. *Diabetes Care,* 2004, vol. 27, 2253-2259
- **DEEDWANIA PC.** Metabolic Syndrome and vascular disease: is nature or nurture leading the new epidemic of cardiovascular disease. *Am Heart Ass Circulation,* 2004, vol. 109, 2-4
- **GOVAERTS SJ ; HERMANS E ; LAMBERT DM.** Comparison of cannabinoid ligand affinities and efficacies in murine tissues and in transfected cells expressing human recombinant cannabinoid receptors. *Eur J Pharm Sci.,* 2004, vol. 23, 233-43
- **GRUNDY SM.** Obesity, Metabolic Syndrome, and cardiovascular disease. *J Clin Endocrinol Metab.,* 2004, vol. 89, 2595-2600
- *Diabetes Atlas,* 2003
- **PHELAN S ; WADDEN TA.** Combining behavioural and pharmacological treatments for obesity. *Obes Res,* 2002, vol. 10, 560-574
- **WHITE MA ; GRILO CM.** Psychometric properties of the Food Craving Inventory among obese patients with binge eating disorder. *Eat Behav,* 2005, vol. 6, 239-245
- **WHITE MA ; WHISENHUNT BL ; WILLIAMSON DA ; GREENWAY FL ; NETEMEYER RG.** Development and validation of the Food-Craving Inventory. *Obes Res,* 2002, vol. 10, 107-114
- *Report of a WHO consultation,* 1999
- **YANOVSKI S.** Sugar and fat: cravings and aversions. *J Nutr,* 2003, vol. 133, 835S-837S
- **ZEPHIER EM ; BALLEW C ; MOKDAD A ; MENDLEIN J ; SMITH C ; YEH JL ; LEE E ; WELTY TK ; HOWARD B.** Intake of nutrients related to cardiovascular disease risk among three groups of American Indians: the Strong Heart Dietary Study. *Prev Med,* 1997, vol. 26, 508-515